# EUROPÄISCHE PATENTSCHRIFT

(11) **EP 1 151 095 B1**
(45) Veröffentlichungstag und Bekanntmachung des Hinweises auf die Patenterteilung: **09.06.2010**
(21) Anmeldenummer: 00901137.0
(22) Anmeldetag: 27.01.2000
(51) Int. Cl.: C12N 15/12, C12N 15/62, C12N 5/10, C07K 14/51, C07K 7/08, A61K 38/18, A61K 31/70, A61P 19/08

(54) **POLYPEPTIDVARIANTEN MIT ERHOEHTER HEPARIN-BINDUNGSFAEHIGKEIT**
POLYPEPTIDE VARIANTS WITH RAISED HEPARIN-BINDING CAPACITY
VARIANTES DE POLYPEPTIDES A CAPACITE AMELIOREE DE LIAISON DE L'HEPARINE

(30) Priorität: 13.02.1999 DE 19906096
(43) Veröffentlichungstag der Anmeldung: 07.11.2001
(73) Patentinhaber: Osteopharma Inc., Osaka (JP)
(72) Erfinder: SEBALD, Walter, D-97074 Würzburg (DE)
(74) Vertreter: Grünecker, Kinkeldey, Stockmair & Schwanhäusser Anwaltssozietät
(86) Internationale Anmeldenummer: PCT/EP2000/000637
(87) Internationale Veröffentlichungsnummer: WO 2000/047736

(56) Entgegenhaltungen:
- EP-A- 0 414 915
- WO-A-91/18558
- WO-A-97/47312
- RUPPERT R. ET AL.: "Human bone morphogenetic protein 2 contains a heparin-binding site which modifies its biological activity" EUR. J. BIOCHEM., Bd. 237, 1996, Seiten 295-302, XP000891887 in der Anmeldung erwähnt
- FROMM J.R. ET AL.: "Differences in the interaction of heparin with arginine and lysine and the importance of thee basic amino acids in the binding of heparin to acidic fibroblast growth factor." ARCHIVES OF BIOCHEMISTRY AND BIOPHYSICS, Bd. 323, Nr. 2, 10. November 1995 (1995-11-10), Seiten 279-287, XP000891918
- ALBERDI E. ET AL.: "Pigment epithelium-derived factor (PEDF) binds to glycosaminoglycans: analysis of the binding site" BIOCHEMISTRY, Bd. 37, 1998, Seiten 10643-10652, XP002135602

## Beschreibung

Die vorliegende Erfindung betrifft Polypeptidvarianten mit erhöhter Heparin-Bindungsfähigkeit. Weiterhin betrifft die vorliegende Erfindung für diese Varianten kodierende Nukleinsäuremoleküle, die Nukleinsäuremoleküle umfassende Wirtszellen und Verfahren zum Herstellen von Polypeptidvarianten und rekombinanten Polypeptidvarianten. Weiterhin betrifft die vorliegende Erfindung die Verwendung der Polypeptidvarianten zur Stimulierung der Chondrogenese, Osteogenese und Wundheilung bzw. zur Behandlung von Entzündung und Krebs. Ferner betrifft die vorliegende Erfindung osteoinduktive Zusammensetzungen, die diese Varianten enthalten.

Zahlreiche biologische Faktoren beeinflussen die Entwicklung und Regeneration von Zellen, Geweben und Organen des menschlichen und tierischen Körpers. Viele dieser Faktoren sind bis heute unbekannt, die von ihnen gesteuerten Prozesse sind noch nicht vollständig verstanden. Auch die Knochenbildung (Osteogenese) ist ein bisher unvollständig verstandener Prozeß; ihr Ablauf ist durch zahlreiche aufeinander folgende Einzelprozesse, wie z. B. Chemotaxis, Mitose und Differenzierung, gekennzeichnet. Chemotaxis bedeutet in diesem Zusammenhang die direkte Einwanderung von Zellen als Antwort auf einen chemischen Gradienten von Signalverbindungen, die aus der unlöslichen demineralisierten Knochenmatrix, die hauptsächlich aus Typ I unlöslichem Collagen besteht, freigesetzt werden. An dieses Typ I unlösliche Collagen bindet Plasma-Fibronektin, welches seinerseits Domänen zur Bindung von Collagen, Fibrin und Heparin aufweist.

An der Steuerung der Osteogenese sind prinzipiell zwei Hauptgruppen von biologisch aktiven Faktoren, die systemisch bzw. die lokal wirkenden Faktoren, beteiligt.

Zur Gruppe der systemisch wirkenden Faktoren gehören beispielsweise die beiden Hormone Parathormon (PTH) und 1,25-Dihydroxy-Vitamin D, die den endogenen Calcium-Spiegel regulieren. Ein weiteres an der Knochenentwicklung beteiligtes Hormon ist Calcitonin, das die Knochenresorption verhindert.

Zu den die Osteogenese regulierenden systemischen Hormone gehören außerdem beispielsweise Östrogene, Androgene, Wachstumshormone, Insulin-like growth factor (IGF), Schilddrüsenhormone und Glucocorticoide.

Zur Gruppe der lokal wirkenden Faktoren gehören
(i) Cytokine, die den Knochenabbau bewirken, wie IL-1, Tumornekrose-Faktor (TNF), IL-6, IL-11 und ODF (Osteoklastendifferenzierungsfaktor, TRANCE);
(ii) Cytokine, die den Knochenabbau verhindern: IL-4, IL-13, IL-18, IFN (Interferon), OPG (Osteoprotegerin) und IL-1ra (Interleukin-1-Rezeptor-Antagonist);
(iii) Kolonie-stimulierende Faktoren: M-CSF (Makrophagen-Kolonie stimulierender Faktor) und GM-CSF (Granulozyten-Makrophagen stimulierender Faktor);
(iv) Prostaglandine, Leukotriene und Stickstoffmonoxid; und
(v) Wachstumsfaktoren: IGF (Insulin-like growth factor; Insulin-artiger Wachstumsfaktor), Proteine aus der DVR (Decapentaplegic-Vg-related)-Familie einschließlich der Proteine der TGF-β (transforming growth factor β; transformierender Wachstumsfaktor β)-Superfamilie, die die Proteine aus der Activin/Inhibin-Familie, MIS (Mullerian Inhibitory Substance; Müllerscher Inhibitor), GDF (growth/differentiation factor; Wachstums/Differenzierungsfaktor), Nodal und Dorsalin umfaßt; FGF (fibroblast growth factor; Fibroblastenwachstumsfaktor), PDGF (platelet-derived growth factor; Blutplättchen-abstammender Wachstumsfaktor) und PTHrP (PTH-related protein; Parathormon-verwandtes Protein).

Im Zusammenhang mit der vorliegenden Erfindung ist die TGF-β Superfamilie von besonderem Interesse.

Die TGF-β Superfamilie umfaßt derzeit mehr als 20 Proteine, wobei die vermuteten Orthologen in verschiedenen Organismen nicht mitgerechnet sind. Neben den TGF-β 's gehören die BMP's (bone morphogenetic proteins), die GDF's (growth differentiation factors) Inhibine/Aktivine und weitere Proteine (Kingsley, 1994) zur Superfamilie. Sie bestehen alle aus zwei Untereinheiten, die fast immer Homodimere aus zwei identischen Monomeren sind und kovalent durch eine Disulfidbrücke verknüpft vorliegen. Kennzeichnendes Merkmal aller bis jetzt strukturell untersuchten Proteine der TGF-β Superfamilie ist das "TGF-β/BMP Gerüst", das aus einem Cystinknoten, einer α-Helix und mindestens vier β-Strängen pro Monomer besteht, und das im Dimer zu einer charakteristischen Anordnung der Monomeren führt (McDonald und Hendrickson, 1993). Die Aminosäuresequenzen können dabei sehr unterschiedlich sein und weisen zum Teil weniger als 40 % identisch besetzte Positionen auf. Besonders variabel sind die "Loop"-Bereiche sowie die N-terminalen Sequenzen. Andererseits ist für alle Faktoren der TGF-β Superfamilie eine erstaunliche Konservierung von Funktion und Struktur in der Stammesgeschichte gezeigt worden. So weisen sie alle ein als "Cystinknoten" bezeichnetes Strukturelement auf, das aus 3 in allen Proteinen der TGF-β Superfamilie konservierten und gleich angeordneten Disulfidbrücken besteht. Besonders gut untersucht sind neben den TGF-β's einzelne Vertreter der BMP's, wie BMP-2 und -7, und Vertreter der GDF's, wie GDF-5, die die Entwicklung und Regenerierung von Knochen und Knorpelgewebe in Gang setzen können. So ist z. B. für BMP-2 gezeigt worden, daß dieses sowohl in heterotopen als auch in orthotopen Implantatlagern osteoinduktive Eigenschaften aufweist.

Die Proteine der TGF-β Superfamilie werden in der Zelle zuerst als großes Proprotein synthetisiert, aus dem dann das reife Protein durch Proteolyse entsteht. Das Proprotein scheint nach einer ArgXXArg Erkennungssequenz gespalten zu werden, so daß eine C-terminale Sequenz von etwa 100 bis 140 Aminosäureresten, die das reife Protein darstellt, freigesetzt wird.

Zur Signaltransduktion binden die Faktoren der TGF-β Superfamilie an die extrazellulären Domänen von 2 Typen membranständiger Rezeptoren ihrer Zielzellen, z. B. induzierbarer Knochenmarksstammzellen. Die Typ 2 Untereinheit enthält im cytoplasmatischen Teil eine Protein-Serinkinase, die nach Ligandenbindung in der Typ 1 Untereinheit Serinreste phosphoryliert. Dadurch wird in der Typ 1 Untereinheit ebenfalls eine Protein-Serinkinase aktiviert, die intrazelluläre Signalproteine wie SMAD's phosphorylieren und aktivieren kann. Es gibt Hinweise, daß sowohl die Typ 1 als auch die Typ 2 Untereinheiten als Dimere existieren. Kürzlich ist die Kristallstruktur der extrazellulären Domäne des Typ 2 Untereinheit des Aktivinrezeptors (Act-RII) aufgeklärt wurden. BMP-2, das zu den besten untersuchten Proteinen der TGF-β Familie gehört, bindet an die Rezeptoruntereinheiten über die strukturell hoch konservierte Domäne nach dem ersten Cystein des Cystinknotens. Die supervariable N-terminale Sequenz vor dem ersten Cystein des Cystinknotens interagiert im BMP-2 nicht direkt mit den Rezeptoruntereinheiten. Die Interaktion von Proteinen der TGF-β Superfamilie mit Typ 1 und Typ 2 Rezeptor Untereinheiten ist zum Teil promiskuitiv und die Spezifitäten überlappen. Es ist noch nicht völlig geklärt, wieweit diese gemeinsame Nutzung von Rezeptor-Untereinheiten oder die Unterschiede im Mechanismus der Rezeptoraktivierung gehen. Aus der gegenwärtigen Literatur geht insbesondere nicht eindeutig hervor, welche Strukturelemente die Spezifität eines Proteins für z.B. eine osteoinduktive Aktivität ausmachen.

Außer mit den Rezeptoruntereinheiten können die Faktoren der TGF-β Superfamilie mit einer Reihe weiterer Proteine interagieren. Dadurch kann die Aktivität der Faktoren moduliert oder gehemmt werden. Fetuin/α2-HS Glykoprotein und ein daraus abgeleitetes Peptid (TRH1) bindet BMP-2 und TGF-β, wobei BMP-2 mit höherer Affinität gebunden wird. Die Bindung kompetiert mit der Bindung an den Rezeptor. Das Noggin-Protein aus Säugetieren bindet BMP-2 mit hoher Affinität in Konkurrenz mit dem Rezeptor. Chordin wirkt, wie in Krallenfrosch-Oozyten gezeigt worden ist, als Hemmstoff für BMP-4. Follistatin bindet mit hoher Affinität an Aktivin und BMP-7. Für BMP-2 ist die Fähigkeit, an Heparin zu binden, nachgewiesen worden.

Das therapeutische Potential der Mitglieder der TGF-β Superfamilie ist aufgrund ihrer physiologischen Bedeutung offensichtlich. Hierbei sind insbesondere rekombinant hergestellte Proteine von Interesse, da sie in großer Menge erhalten werden können. Darüber hinaus sind die für sie kodierenden Nukleinsäuren potentielle Gen-Therapeutika.

Somit besteht ein generelles Interesse an Mitgliedern der TGF-β Superfamilie und Varianten mit veränderten biologischen Eigenschaften. Kübler et al. (1999) beschreiben ein BMP-Analogon EHBMP-2, dessen Primärstruktur von der des natürlicherweise vorkommenden humanen BMP-2 dadurch abweicht, daß die mutmaßlich für die starke Heparin-Bindung von BMP-2 verantwortlichen ersten 12 Aminosäuren durch die ersten 13 Aminosäuren von humanem Interleukin-2 ersetzt sind. Das genetisch veränderte BMP-2-Analogon wurde rekombinant in *E. coli* exprimiert. EHBMP-2 weist eine vernachlässigbare Affinität zu Heparin und eine höhere biologische Aktivität in verschiedenen Zellkulturen, d.h. *in vitro,* auf. Beim Vergleich der *in vivo*-Aktivität der Variante mit natürlichem BMP-2 zeigte sich jedoch, daß in der Maus durch BMP-2-Konzentrationen ab 4 µg in annähernd allen Proben eine heterotope Knocheninduktion erzeugt wurde, wohingegen zur Erzielung desselben Effekts eine Menge von ca. 40 µg EHBMP-2 benötigt wurde. Weiterhin wurde beobachtet, daß das resultierende Ausmaß der Knochenneubildung bei gleichen Proteinkonzentrationen mit dem natürlichen BMP-2 signifikant größer war als bei seinem BMP-Analogon EHBMP-2. Ruppert et al (1996) beschreibt eine Heparin-Bindungsstelle im N-terminalen Bereich von hBMP-2. Ferner wird eine hBMP-2-Variante beschrieben, die keine Bindung an Heparin, jedoch eine erhöhte spezifische Aktivität aufweist.

Die der vorliegenden Erfindung zugrunde liegende Aufgabe besteht somit darin, weitere Polpeptidvarianten bereitzustellen, deren *in vivo* Wirksamkeit derjenigen des Wildtyps entspricht oder erhöht ist. Eine weitere Aufgabe liegt in der Bereitstellung von für die Polpeptidvarianten kodierenden Nukleinsäuren und Vektoren und Wirtszellen, die diese Nukleinsäuren enthalten. Weiterhin sollen Verfahren zum Herstellen der Polypeptidvarianten bereitgestellt werden. Schließlich sollen pharmazeutische Zusammensetzungen, die diese Polypeptidvarianten enthalten, und deren Verwendung bereitgestellt werden.

Diese Aufgabe wird erfindungsgemäß gelöst durch eine Polypeptidvariante mit erhöhter Heparin-Bindungsfähigkeit, die dadurch gekennzeichnet ist, dass die Polypeptidvariante eine erhöhte Heparinbindungsfähigkeit aufweist, wobei an die Aminosäuresequenz des Polypeptids, ausgewählt aus Mitgliedern der TGFβ-Superfamilie, wenigstens ein Oligopeptids, ausgewählt aus Mitgliedern der TGFβ-Superfamilie, wenigstens ein Oligopeptid addiert und/oder inseriert ist und/oder wenigstens eine natürlicherweise in der Aminosäuresequenz des Polypeptids vorkommende Oligopeptidsequenz durch ein Oligopeptid substituiert ist, wobei das Oligopeptid die Aminosäuresequenz RKRA (SEQ ID NO: 3) oder RKRAKHKQ (SEQ ID NO: 4) umfasst.

Ferner ist hierin eine Polypeptidvariante beschrieben mit erhöhte Heparin-Bindungsfähigkeit die dadurch gekennzeichnet ist, dass
(i) an die Aminosäuresequenz eines Polypeptides mit geringer Heparin-Bindungsfähigkeit wenigstens ein Oligopeptid, umfassend die Aminosäuresequenz X₁ X₂ X₃ X₄ X₅ X₆, addiert ist und/oder,
(ii) in die Aminosäuresequenz eines Polypeptides wenigstens ein Oligopeptid, umfassend die Aminosäuresequenz X₁X₂X₃X₄X₅X₆, inseriert ist und/oder
(iii) wenigstens eine natürlicherweise in der Aminosäuresequenz eines Polypeptids vorkommende Oligopeptidsequenz durch ein Oligopeptid, umfassend eine Aminosäuresequenz X₁X2X₃X₄X₅X₆, substituiert ist,
wobei
X₁ = K, R oder H;
X₂ = K, R oder H;
X₃ = K, R, H oder keine Aminosäure;
X₄ = kein K, R, H, sonst beliebige Aminosäure;
X₅ = kein K, R, H, sonst beliebige oder keine Aminosäure;
X₆ = kein K, R, H, sonst beliebige oder keine Aminosäure, bedeutet (SEQ ID No: 1)
oder
X₁ = K, R oder H;
X₂ = kein K, R, H, sonst beliebige Aminosäure;
X₃ = K, R oder H;
X₄ = kein K, R, H, sonst beliebige Aminosäure;
X₅ = kein K, R, H, sonst beliebige oder keine Aminosäure;
X₆ = kein K, R, H, sonst beliebige oder keine Aminosäure (SEQ ID No: 2),
bedeutet.

Im nachfolgenden werden einige Begriffe näher erläutert, um klarzustellen, wie sie im Zusammenhang der vorliegenden Anmeldung verstanden werden sollen.

Der Begriff "Polypeptid", so, wie er nachfolgend in der Beschreibung verwendet wird, umfaßt aus 5 oder mehr Aminosäuren zusammengesetzte Peptide oder Proteine, die wenigstens eine biologische Aktivität aufweisen. Der Begriff umfaßt ferner biologisch aktive Fragmente der Polypeptide, Mutanten und Fusionsproteine davon.

"Polypeptidvariante mit erhöhter Heparin-Bindungsfähigkeit" bedeutet, daß die Polypeptidvariante eine Heparin-Bindungsfähigkeit aufweist, die gegenüber der Heparin-Bindungsfähigkeit des nicht veränderten Polypeptides erhöht ist. Die Heparin-Bindungsfähigkeit allgemein kann beispielsweise durch Plasmonresonanz-Analyse mit Hilfe eines Heparin-beschichteten Trägers gemessen werden. Die einzelnen Versuchsbedingungen sind beispielsweise beschrieben in Ruppert et al., 1996.

Unter der *in vivo* Wirksamkeit wird das Ausmaß der bestimmungsgemäßen Wirkung am Zielort verstanden. Die *in vivo* Wirksamkeit wird bestimmt durch die biologische Aktivität des Proteins in Verbindung mit der Verfügbarkeit des Proteins am Zielort. Sie wird letztendlich gemessen als Wirkung am Zielort. Für die Messung der Wirkung kann auf etablierte Verfahren zurückgegriffen werden. Im Fall von BMP-2 Varianten hat sich die Induktion der ektopen Knochenbildung, beschrieben in Kübler & Urist, 1991 und Kübler et al., 1999, bewährt.

Als "Wachstumsfaktor" wird ein biologisch aktives Polypeptid bezeichnet, das das Wachstum und/oder die Differenzierung von Zellen moduliert.

Insofern nachfolgend auf bestimmte Polypeptide eingegangen wird, so ist die Aminosäuresequenz beispielsweise erhältlich aus der öffentlich zugänglichen Datenbank Entrez (z.Zt.: http://www.ncbi.nlm.nih.gov/Entrez/).

Der dem Fachmann bekannte Ausdruck "Homologie" bezeichnet den Grad der Verwandtschaft zwischen zwei oder mehr Polypeptiden, der durch die Übereinstimmung zwischen den Aminosäuresequenzen mittels bekannter Verfahren, z. B. computergestützter Sequenzvergleiche (Basic local alignment search tool, S.F. Altschul et al., J. Mol. Biol. 215 (1990), 403-410) bestimmt wird. Der Prozentsatz der "Homologie" ergibt sich aus dem Prozentsatz identischer Bereiche in zwei oder mehr Sequenzen unter Berücksichtigung von Lücken oder anderen Sequenzbesonderheiten. In der Regel werden spezielle Computerprogramme mit Algorithmen eingesetzt, die den besonderen Anforderungen Rechung tragen.

Bevorzugte Verfahren zur Bestimmung der Homologie erzeugen zunächst die größte Übereinstimmung zwischen den untersuchten Sequenzen. Computerprogramme zur Bestimmung der Homologie zwischen zwei Sequenzen umfassen, sind jedoch nicht eingeschränkt auf das GCG Programmpaket, einschließlich GAP (Devereux, J., et al., Nucleic Acids Research 12 (12) : 387 (1984); Genetics Computer Group University of Wisconsin, Madison, (WI)); BLASTP, BLASTIN, und FASTA (Altschul, S. et al., J. Mol. Biol. 215:403-410) (1990)). Das BLASTX Programm kann vom National Centre for Biotechnology Information (NCBI) und aus weiteren Quellen bezogen werden (BLAST Handbuch, Altschul S., et al., NCB NLM NIH Bethesda MD 20894; Altschul, S., et al., Mol. Bio. 215:403-410 (1990)). Auch der bekannte Smith Waterman-Algorithmus kann zur Bestimmung von Homologien verwendet werden.

Bevorzugte Parameter für den Aminosäuresequenz-Vergleich umfassen die nachstehenden:
- Algorithmus:: Needleman und Wunsch, J. Mol. Biol 48:443-453 (1970)
- Vergleichsmatrix:: BLOSUM 62 aus Henikoff und Henikoff, PNAS USA 89(1992), 10915-10919
- Lücken-Wert (Gap Penalty):: 12
- Lückenlängen-Wert: (Gap Length Penalty):: 4
Homologie-Schwellenwert (Threshold of Similarity): 0

Das GAP-Programm ist auch zur Verwendung mit den vorstehenden Parametern geeignet. Die vorstehenden Parameter sind die Fehler-Parameter (default parameters) für Aminosäuresequenz-Vergleiche, wobei Lücken an den Enden den Homologie-Wert nicht verringern. Bei sehr kurzen Sequenzen im Vergleich zur Referenzsequenz kann es weiterhin notwendig sein, den Erwartungswert auf bis zu 100.000 (expectation value) zu erhöhen und gegebenenfalls die Wortlänge (word size) auf bis zu 2 zu verkleinern.

Weitere beispielhafte Algorithmen, Lücken-Öffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermittelte Übereinstimmung von 60 % wird im Rahmen dieser Anmeldung als 60 % Homologie bezeichnet. Entsprechendes gilt für höhere Homologiegrade.

"His-Tag" bedeutet eine Sequenz von wenigstens 6 Histidin-Aminosäuren, die durch entsprechende Klonierung und Fusion mit einer exprimierbaren Sequenz zu einem Fusionsprotein mit wenigstens 6 His-Resten am NH₂-Terminus führt, das leicht durch Komplexierung mit einer Ni²⁺-Säule aufgereinigt werden kann.

Unter einem "heterologen Gen" ist der kodierende Bereich eines Strukturgens zu verstehen, der entweder nicht unter der Kontrolle des eigenen (homologen) Promotors oder nicht in dem Organismus, aus dem er abgeleitet ist, oder weder unter der Kontrolle des eigenen Promotors noch im ursprünglichen Organismus exprimiert wird.

"Klonierung" soll alle im Stand der Technik bekannten Klonierungsmethoden umfassen, die hier zum Einsatz kommen könnten, die jedoch nicht alle im einzelnen beschrieben werden, weil sie zum selbstverständlichen Handwerkszeug des Fachmanns gehören.

Unter "rekombinanter Expression in einer geeigneten Wirtszelle" sollen alle im Stand der Technik bekannten Expressionsmethoden in bekannten Expressionssystemen verstanden werden, die hier zum Einsatz kommen könnten, jedoch nicht alle im einzelnen beschrieben werden, weil sie zum selbstverständlichen Handwerkszeug des Fachmanns gehören.

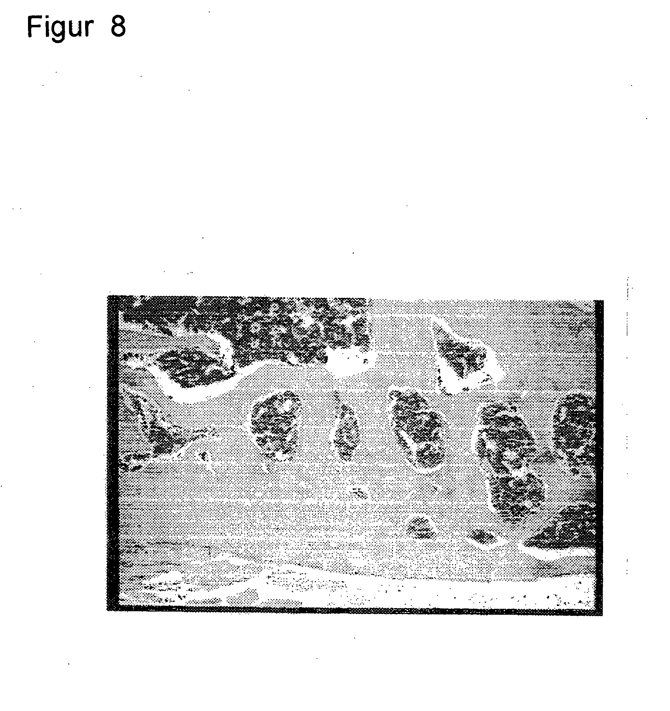

Überraschenderweise wurde nun gefunden, daß eine erfindungsgemäße Polypeptidvariante eine höhere Heparin-Bindungsfähigkeit gegenüber dem nicht veränderten Polypeptid dadurch aufweist, daß wenigstens ein Oligopeptid, das eine erfindungsgemäße Aminosäuresequenz umfaßt, an das Polypeptid addiert und/oder in das Polypeptid inseriert wird und/oder einige der Aminosäuren als Polypeptide ersetzt. Es konnte gezeigt werden, daß im Vergleich zum nicht veränderten Polypeptid die Menge der an Heparin gebundenen Varianten größer ist und daß außerdem die Abdissoziierung der Varianten von Heparin verringert ist. Da Heparin-ähnliche Strukturen integrale Bestandteile der Knochenstruktur sind, weisen die erfindungsgemäßen Polypeptidvarianten auch eine erhöhte Bindungsfähigkeit für Knochenstrukturen auf. Diese Eigenschaft ist besonders wichtig für regenerative Vorgänge in der Knochenstruktur, wobei in diesem Fall die beteiligten Polypeptide an der Knochenmorphogenese (Osteogenese) mitwirken. Es konnte dabei gezeigt werden, daß die erfindungsgemäßen Polypeptidvarianten die Heparinbindungsfähigkeit um den Faktor 30, mindestens aber den Faktor 10 steigern konnten. Die erhöhte Heparinbindungsfähigkeit wiederum führte zu einer dramatisch gesteigerten *in vivo* Wirksamkeit, wie z. B. durch die Figur 8 belegt.

Wie erwähnt, wird die Wirkung der an der Osteogenese beteiligten Polypeptide durch Rezeptoren vermittelt, die u.a. auf der Oberfläche von induzierbaren Knochenmarksstroma-Stammzellen vorkommen. Diese haben die Fähigkeit, nach Einwirkung eines induktiven Signals von morphogenetischen Proteinen oder demineralisierter Knochenmatrix, Knochenzellen auszubilden. Es wird angenommen, ohne an diese Theorie gebunden sein zu wollen, daß die Erhöhung der Heparin-Bindungsfähigkeit von Polypeptiden, insbesondere von osteoinduktiven Polypeptiden, zur Erhöhung der lokalen Konzentration von osteogenetischen Proteinen in unmittelbarer Nähe des Ortes der Knochenneubildung führt.

Da jedoch Heparin mit dem zellulären Rezeptor des Polypeptides um die Polypeptidvariante konkurriert, ist zunächst davon auszugehen, daß die Erhöhung der Heparin-Bindungsfähigkeit der Polypeptidvariante zu einer Abnahme der für den Polypeptidspezifischen Rezeptor verfügbaren Polypeptid-Menge und damit zu einer verminderten Signaltransduktion führt.

Überraschenderweise wurde nun gefunden, daß die biologische Verfügbarkeit der Polypeptidvariante bei gleichzeitiger Erhöhung der Heparin-Bindungsfähigkeit nicht beeinträchtigt ist, daß vielmehr die erhöhte Heparin-Bindungsfähigkeit mit einer Erhöhung der lokalen Konzentration von für den spezifischen Rezeptor verfügbarem Polypeptid korreliert.

Weiterhin wurde überraschenderweise gefunden, daß die Qualität des durch die Polypeptidvariante *in vivo* gebildeten Knochens gegenüber derjenigen, die durch das nicht veränderte Polypeptid gebildet wurde, erhöht ist.

In einer bevorzugten Ausführungsform ist die erfindungsgemäße Aminosäuresequenz an den N-Terminus des reifen Polypeptids addiert und/oder durch Substitution bzw. Insertion in den N-terminalen Bereich des Polypeptides eingefügt. Der N-terminale Bereich bedeutet hierbei die ersten 20, vorzugsweise die ersten fünf, N-terminalen Aminosäuren.

Sofern das Polypeptid zur TGF-β Superfamilie gehört, sind die reifen Polypeptide, z. B. ausgehend von BMP-2, wie folgt ermittelbar: Eine Suche in der "Swiss-Prot" Datenbank (derzeit http://expasy.hcuge.ch/cgi-bin/sprot-search-de) mit dem Suchwort "BMP-2" ergibt unter der Nummer P12643 die Aminosäuresequenz des gesamten Proproteins von humanem BMP-2. Daraus läßt sich unter "bone morphogenetic protein 2" die Sequenz für das reife Protein auswählen. Sie entspricht den Aminosäureresten 283-396. Um die anderen Proteine der TGF-β Superfamilie zu identifzieren, wird diese Sequenz 283-396 unter "BLAST2" bei EMBnet-CH (Lausanne, Schweiz) eingereicht. Dort wird sie gegen die Datenbanken Swiss-Prot + TrEMBL + TrEMBL_New mit Hilfe des Programms "blastp" abgesucht. Dabei wird die Vergleichsmatrix Blosum 62 verwendet, und es werden die 250 am nächsten verwandten Proteine ermittelt. Das Programm liefert die entsprechenden Identitäts-Nummern der Swiss-Prot Datenbank, unter denen sich die Aminosäuresequenzen der gesamten Proproteine aufrufen lassen. Die Sequenzabschnitte für die reifen Proteine sind jeweils angegeben. Sie ließen sich sonst auch ableiten, da die reifen Proteine gewöhnlich nach der Erkennungssequenz RXXR abgespalten werden (X bedeutet hier eine beliebige Aminosäure).

Alle Polypeptide der TGF-β Superfamilie sind, wie schon erwähnt, durch ein Strukturelement gekennzeichnet, das als "Cystinknoten" bezeichnet wird (McDonald und Hendrickson, 1993). Gehört das Polypeptid zu dieser Superfamilie, so gilt der gesamte Bereich vor dem Cystinknoten als N-terminaler Bereich, und die erfindungsgemäße Aminosäuresequenz wird vorzugsweise vor dem Cystinknoten eingefügt.

In einer besonders bevorzugten Ausführungsform ist das Oligopeptid, das die erfindungsgemäße Aminosäuresequenz umfaßt, ein bis viermal durch Addition, Insertion und/oder Substitution in das Polypeptid eingefügt, wobei jeweils ein oder mehrere Kopien des Oligopeptids an einer oder mehreren Positionen in das Polypeptid eingefügt werden können.

In einer besonders bevorzugten Ausführungsform hat das Oligopeptid die Sequenz RKRA (SEQ ID No. 3) oder die Sequenz RKRAKHKQ (SEQ ID No. 4).

Weiterhin ist bevorzugt, daß die Polypeptidvariante eine für die rekombinante Expression relevante Sequenz am N-Terminus umfaßt, wobei die für die rekombinante Expression relevante Sequenz M oder MZ ist, und M Methionin und Z eine oder mehrere beliebige Aminosäuren bedeutet. MZ kann beispielsweise eine Signalsequenz darstellen, wie sie für zahlreiche prokaryontische und eukaryontische Proteine dem Fachmann bekannt ist. Es kann sich dabei um auf das jeweilige Expressionssystem zugeschnittene Signalsequenzen handeln oder aber um die "homologen" Signalsequenzen, d.h., die zu dem Protein natürlicherweise gehörenden Signalsequenzen, sowie schließlich um eine Kombination einer beliebigen Signalsequenz mit gezielt eingefügten Proteaseschnittstellen, etc.

Ferner ist bevorzugt, daß die Polypeptidvariante ein His-Tag umfaßt. Das His-Tag am NH₂-Terminus der Polypeptidvariante erleichtert die Aufreinigung des Proteins wesentlich, da diese Aufreinigung an einer Nickel-Säule durch Chelat-Bildung durchgeführt werden kann.

Das der Polypeptidvariante zugrundeliegende Polypeptid weist eine biologische Aktivität auf. Hierbei sind grundsätzlich sämtliche biologischen Aktivitäten von Interesse, bei denen die Wirksamkeit eines Polypeptides mit biologischer Aktivität durch das Wegdiffundieren von negativ geladenen intra- und extrazellulären Strukturen limitiert ist. Vorzugsweise sind diese negativ geladenen Strukturen Strukturen der extrazellulären Matrix, die aufgrund ihres Anteils an Proteoglykanen und Glykosaminoglykanen, wie Heparansulfat, Chondroitinsulfat, Keratansulfat und Dermatansulfat, negativ geladen sind. In einer bevorzugten Ausführungsform reguliert die biologische Aktivität die Entwicklung bzw. Differenzierung von Zellen, Geweben und Organen des menschlichen oder tierischen Körpers.

Besonders bevorzugt ist hierbei, daß das der Polypeptidvariante zugrundeliegende Polypeptid die Knochenbildung reguliert (osteogenetische Aktivität). Die osteogenetische Aktivität kann hierbei beispielsweise durch den Wachstumsfaktor-abhängigen Einbau von ³⁵SO₄ in Proteoglykan-Strukturen von Gliedmaßenknospen aus Hühnerembryonen bestimmt werden. Sinnvollerweise wird dazu ein Konzentrationsbereich für das BMP-Protein gewählt, der es ermöglicht, sowohl die maximale Zellantwort als auch die EC₅₀ (Konzentration, bei der 50% des maximalen Einbaus erreicht werden) zu bestimmen. Die Assay-Bedingungen sind angegeben in Ruppert et al., 1996.

Alternativ kann die myoblastäre Maus-Zelllinie C2C12 verwendet werden, um die BMP-abhängige Induktion der Alkalischen Phosphatase zu bestimmen (Katagiri et al., 1994) Auch mit diesem Test können die maximale Zellantwort und die EC₅₀ bestimmt werden. Bevorzugt ist das Polypeptid aus der Hormone, Cytokine und Wachstumsfaktoren umfassenden Gruppe ausgewählt. Hierbei sind besonders bevorzugt:
Parathormon (PTH); Calcitonin; Wachstumshormon; Insulin-like growth factor (IGF); Cytokine, die den Knochenabbau bewirken, wie IL-1, Tumornekrose-Faktor (TNF), IL-6, IL-11 und ODF (Osteoklastendifferenzierungsfaktor, TRANCE); Cytokine, die den Knochenabbau verhindern: IL-4, IL-13, IL-18, IFN (Interferon), OPG (Osteoprotegerin) und IL-1ra (Interleukin-1-Rezeptor-Antagonist); Kolonie-stimulierende Faktoren: M-CSF (Makrophagen-Kolonie stimulierender Faktor) und GM-CSF (Granulozyten-Makrophagen stimulierender Faktor); und Wachstumsfaktoren: IGF (Insulin-like growth factor; Insulin-artiger Wachstumsfaktor); Proteine aus der DVR-Familie einschließlich der Proteine aus der TGF-β (transforming growth factor β; transformierender Wachstumsfaktor β)-Superfamilie, die wiederum die Activin/Inhibin-Familie, MIS (Mullerian Inhibitory Substance; Müllerscher Inhibitor), die GDF (growth/differentiation factor; Wachstums-/Differenzierungsfaktor)-Familie, Nodal und Dorsalin umfaßt; FGF (fibroblast growth factor; Fibroblastenwachstumsfaktor); PDGF (platelet-derived growth factor; Blutplättchen-abstammender Wachstumsfaktor) und PTHrP (PTH-related protein; Parathormon-verwandtes Protein).

Besonders bevorzugt umfaßt das Polypeptid Mitglieder der TGF β-Superfamilie, Activin/Inhibin-Familie, MIS, GDF-Familie, Nodal und Dorsalin, sowie Mitglieder der BMP-Familie, insbesondere BMP-2, BMP-4, BMP-5, BMP-6, BMP-7/OP-1 oder BMP-8/OP-2, sowie BMP-9, BMP-10, BMP-11, BMP-12, BMP-13, BMP-14 und BMP-15.

Die vorstehende Auswahl von Wachstumsfaktoren ist erfindungsgemäß von besonderem Interesse, da die Beteiligung dieser Faktoren an der Osteogenese bekannt ist (Reddi, 1998); die Verwendung der erfindungsgemäßen Varianten dieser Faktoren führt zu einer erhöhten Heparin-Bindungsfähigkeit unter Beibehaltung der osteoinduktiven Eigenschaften.

Sofern das Polypeptid der TGF-β Superfamilie angehört und somit die vorstehend erläuterte "Cystinknoten"-Struktur aufweist, wird die erfindungsgemäße Aminosäuresequenz bevorzugt vor den Cystinknoten in die Polypeptidsequenz eingefügt. Besonders bevorzugte Positionen sind beispielsweise bei BMP-2 die Positionen zwischen den Aminosäureresten 2 und 3, 6 und 7, 10 und 11 sowie 13 und 14 des reifen Proteins. Aufgrund der Homologie zwischen den Mitgliedern der TGF-β Familie, insbesondere der konservierten Anordnung der Cysteine, kann der Fachmann den genannten Proteinen entsprechende Positionen in anderen TGF-β Familienmitgliedern bestimmen.

Das Polypeptid kann ferner ein Hormon, ein Cytokin oder ein Wachstumsfaktor sein, der eine Addition, Substitution, Insertion, Inversion und/ oder Deletionen aufweist, wobei das die Addition, Substitution, Insertion, Inversion und/oder Deletion aufweisende Polypeptid wenigstens 10 %, vorzugsweise wenigstens 50 % und insbesondere wenigstens 90 % der biologischen Aktivität, im Fall osteogenetischer Polypeptide der osteogenetischen Aktivität des ursprünglichen Polypeptids aufweist. Die biologische Aktivität kann hierbei allgemein mit jedem dem Fachmann bekannten Verfahren zur Messung der biologischen Aktivität des Polypeptides bestimmt werden.

In einer weiteren bevorzugten Ausführungsform sind die durch Addition, Substitution, Insertion, Inversion oder Deletion erhaltenen Polypeptide wenigstens 50 %, vorzugsweise 75 % und insbesondere 90 % homolog zur Aminosäuresequenz des vollständigen ursprünglichen Polypeptids. Dabei kann eine wenigstens 10%ige biologische Aktivität mit einer mindestens 50%igen, mindestens 75%igen oder mindestens 90%igen Homologie korrelieren. Gleiches gilt für eine wenigstens 50%ige oder wenigstens 90%ige biologische Aktivität.

Besonders bevorzugt sind Polypeptidvarianten mit der Aminosäuresequenz SEQ ID No 5 (T3) oder SEQ ID No 6 (T4). Diese Polypeptidvarianten entsprechen den in den Beispielen verwendeten Polypeptidvarianten, die eine erhöhte Wirksamkeit bei gleicher Konzentration und eine verbesserte Qualität des induzierten Knochens zeigen, wobei unter verbesserter Qualität insbesondere eine dichtere Knochenmatrix und damit eine erhöhte biomechanische Belastbarkeit zu verstehen sind.

In einer weiteren bevorzugten Ausführungsform wird die erfindungsgemäß hergestellte Polypeptidvariante modifiziert. Die Modifikationen umfassen hierbei die Di-, Oligo- und Polymerisierung des monomeren Ausgangsprodukts, beispielsweise durch Quervernetzung, z.B. durch Dicyclohexylcarbodiimid oder Pegylierung oder Assoziation (self assembly). Die somit hergestellten Di-, Oligo- und Polymere können voneinander beispielsweise durch Gelfiltration abgetrennt werden. Weitere Modifikationen umfassen Seitenketten-Modifikationen, beispielsweise von ε-Amino-Lysin-Resten der Polypeptidvariante, oder Amino- bzw. Carboxy-terminale Modifikationen. Schließlich umfaßt der Begriff Modifikationen posttranslationale Ereignisse, z.B. die Glykosylierung oder die partielle oder vollständige Deglykosylierung des Proteins.

Die Erfindung stellt ferner Nukleinsäuremoleküle bereit, die eine für eine erfindungsgemäße Polypeptidvariante kodierende Nukleinsäuresequenz umfassen.

Die im erfindungsgemäßen Nukleinsäuremolekül enthaltene Nukleinsäuresequenz kann von einer genomischen DNA, cDNA oder synthetischen DNA abgeleitet sein, wobei unter synthetischen DNA-Sequenzen auch solche verstanden werden, die modifizierte Internukleosid-Bindungen enthalten. Weiterhin kann es sich bei den Nukleinsäuresequenzen um RNA-Sequenzen handeln, was z.B. für die Expression mittels rekombinanter RNA-Vektorsysteme erforderlich sein kann.

Bevorzugte Nukleinsäuremoleküle umfassen eine für eine der Polypeptidvarianten T3 oder T4 kodierende Nukleinsäure. Beispiele solcher Nukleinsäuren sind im Sequenzprotokoll unter SEQ ID No. 7 (T3) und SEQ ID No. 8 (T4) angegeben. Es versteht sich von selbst, daß anstelle der in SEQ ID No. 7 oder SEQ ID No. 8 angegebenen Nukleinsäuresequenzen solche, die auf einer Degeneration des genetischen Codes beruhen, ebenfalls verwendet werden können. Bevorzugt sind in diesem Zusammenhang Nukleinsäuresequenzen, die im Hinblick auf die Expression in einem bestimmten Wirtsorganismus eine an den Codon-Gebrauch dieses Wirtsorganismus adaptierte Codon-Auswahl aufweisen. Zu den vorgenannten Sequenzen komplementäre Sequenzen sind von der Erfindung ebenfalls umfaßt.

In einer bevorzugten Ausführungsform umfaßt das erfindungsgemäße Nukleinsäuremolekül einen zur Expression geeigneten Promotor, wobei die Nukleinsäuresequenz unter der Kontrolle des Promotors steht. Die Wahl des Promotors hängt vom zur Expression verwendeten Expressionssystem ab. Generell sind induzierbare Promotoren, wie z.B. der Metallothionein-Promotor, bevorzugt, jedoch sind auch konstitutive Promotoren möglich.

In einer weiteren bevorzugten Ausführungsform umfaßt das Nukleinsäuremolekül wenigstens einen Teil eines Vektors, insbesondere regulatorische Regionen, wobei der Vektor ausgewählt sein kann aus Bakteriophagen wie λ-Derivaten, Adenoviren, Vacciniaviren, Baculoviren, SV40-Virus, Retroviren, Plasmiden, wie Ti-Plasmide von *Agrobacterium tumefaciens,* YAC-Vektoren und BAC-Vektoren. Bevorzugte Vektoren sind pR^{TS}pRC 109 (Weigel et al., 1989) und pRBSIIPN₂₅x/o (Stueber, 1994).

Ferner stellt die Erfindung Wirtszellen bereit, die das Nukleinsäuremolekül enthalten und die zur Expression des Nukleinsäuremoleküls geeignet sind. Im Stand der Technik sind zahlreiche prokaryontische und eukaryontische Expressionssysteme bekannt, wobei die Wirtszellen beispielsweise ausgewählt sind aus prokaryontischen Zellen, wie *E. coli* oder *B. subtilis,* aus eukaryontischen Zellen, wie Hefezellen, Pflanzenzellen, Insektenzellen und Säugerzellen, z.B. CHO-Zellen, COS-Zellen oder HeLa-Zellen, sowie Derivaten davon. Im Stand der Technik sind beispielsweise bestimmte CHO-Produktlonslinien bekannt, deren Glykosylierungsmuster im Vergleich zu CHO-Zellen verändert sind. Die durch die Verwendung Glykosylierung-effizienter oder Glykosylierung-verringerter Wirtszellen erhaltenen Polypeptidvarianten verfügen möglicherweise über eine veränderte räumliche Struktur, die gegebenenfalls zu einer erhöhten biologischen Aktivität gegenüber der glykosylierten Polypeptidvariante führen kann, sofern das Polypeptid biologische Aktivität aufweist.

Gegenstand der vorliegenden Erfindung ist ein verfahren zum Herstellen einer Polpeptidvariante mit erhöhter Heparin-Bindungsfähigkeit gemäss Anspruch 1, umfassend das Addieren wenigstens eines Oligopeptides, das eine Aminosäuresequenz enthält, die ausgewählt ist aus: SEQ ID NO: 3 und SEQ ID NO: 4, an die Aminosäuresequenz eines Polpeptides;
und/oder das Inserieren wenigstens eines Oligopeptides, das eine Aminosäuresequenz enthält, die ausgewählt ist aus: SEQ ID NO: 3 und SEQ ID NO: 4 eines Polpeptides; und/oder das Substituieren wenigstens einer natürlicherweise in der Aminosäuresequenz eines Polpeptids vorkommenden Oligopeptidsequenz durch ein Oligopeptid, das eine Aminosäuresequenz enthält, die ausgewählt ist aus: SEQ ID NO: 3 und SEQ ID NO: 4.

Das Verfahren gemäß der Erfindung umfasst:
a) *in vitro* Mutagenese einer für ein Polypeptid kodierenden Nukleinsäure, so dass (i) an die für das Polpeptid kodierende Nukleinsäure wenigstens eine Nukleinsäure addiert wird, die für ein Oligopeptid kodiert, das eine Aminosäuresequenz, ausgewählt aus SEQ ID NOS: 3 und 4, enthält; und/oder (ii) in die für das Polypeptid kodierende Nukleinsäure wenigstens eine Nukleinsäure inseriert wird, die für ein Oligopeptid kodiert, das eine Aminosäuresequenz, ausgewählt aus SEQ ID NOS: 3 und 4, enthält; und/oder (iii) wenigstens eine natürlicherweise in der für das Polypeptid kodierenden Nukleinsäure vorkommende Nukleinsäuresequenz durch eine Nukleinsäuresequenz substituiert wird, die für ein Oligopeptid kodiert, das eine Aminosäuresequenz, ausgewählt aus: SEQ ID NO:3 und SEQ ID NO: 4, enthält;
b) Einklonieren der mutierten Nukleinsäure in einen geeigneten Espressionsvektor;
c) Transformieren/Transfizieren einer geeigneter Wirtszelle mit dem erhaltenen Expressionsvektor;
d) Kultivieren der transformierten/transfizierten Wirtszelle unter zur Expression geeigneten Bedingungen;
e) Isolierung und gegebenenfalls Renaturieren der exprimierten Polypeptidvariante.

Ferner ist hierin ein Verfahren zum Herstellen einer Polpeptidvariante mit erhöhter Heparin-Bindungsfähigkeit beschrieben, umfassend das Addieren wenigstens eines Oligopeptides, enthaltend eine Aminosäuresequenz, die aus: SEQ ID NO: 1 und SEQ ID NO: 2 ausgewählt ist, an die Aminosäuresequenz eines Polypeptids, und/oder das Inserieren wenigstens eines Oligopeptids, das eine Aminosäuresequenz enthält, die aus: SEQ ID No: 1 und SEQ No: 2 ausgewählt ist, in die Aminosäuresequenz des Polypeptids; und/oder das Substituieren wenigstens einer natürlicherweise in der Aminosäuresequenz vorkommenden Oligopeptidsequenz durch ein Oligopeptid, das eine Aminosäuresequenz enthält, die aus: SEQ ID No: 1 und SEQ No: 2 ausgewählt ist.

Das Verfahren kann durch chemische Partial- oder Total-Synthese, unter Verwendung der bekannten Merrifield-Synthese oder durch enzymatische Synthese durchgeführt werden. Weiterhin kann das Verfahren gentechnologisch, d.h. durch rekombinante Expression durchgeführt werden. Die Erfindung umfaßt ferner auch die Kombination aus chemischen/enzymatischen und gentechnologischen Verfahren.

In einer bevorzugten Form umfaßt das Verfahren:
a) *in vitro* Mutagenese einer für ein Polypeptid kodierenden Nukleinsäure, so daß (i) an die für das Polypeptid kodierende Nukleinsäure wenigstens eine Nukleinsäure addiert wird, die für ein Oligopeptid kodiert, das eine Aminosäuresequenz enthält, die aus SEQ ID No: 1 und SEQ ID No: 2 ausgewählt ist; und/oder (ii) in die für das Polypeptid kodierende Nukleinsäure wenigstens eine Nukleinsäure inseriert wird, die für ein Oligopeptid kodiert, das die Aminosäuresequenz, ausgewählt aus SEQ ID No: 1 und 2, enthält und/oder (iii) wenigstens eine natürlicherweise in der für das Polypeptid kodierenden Nukleinsäure vorkommende Nukleinsäuresequenz durch eine Nukleinsäuresequenz substituiert wird, die für ein Oligopeptid kodiert, das eine Aminosäuresequenz enthält, die aus: SEQ ID No: 1 und SEQ ID No: 2 ausgewählt ist;
b) Einklonieren der mutierten Nukleinsäure in einen geeigneten Expressionsvektor;
c) Transformieren/Transfizieren einer geeigneten Wirtszelle mit dem erhaltenen Expressionsvektor;
d) Kultivieren der transformierten/transfizierten Wirtszelle unter zur Expression geeigneten Bedingungen;
e) Isolieren und gegebenenfalls Renaturieren der Expressionsprodukte.

Dem Fachmann sind zahlreiche Verfahren zur Expression von DNA-Sequenzen bekannt; vgl. Recombinant Gene Expression Protocols in Methods in Molecular Biology, engl. Text, Band 62, Humana Press Totowa, New Jersey (1995). Die Expression kann sowohl konstitutiv als auch induzierbar sein, wobei Induktoren wie beispielsweise IPTG und Zn²⁺ dem Fachmann bekannt sind.

Die der Mutagenese zuzuführenden Nukleinsäuresequenzen, die für ein Polypeptid mit biologischer Aktivität kodieren, sind beispielsweise durch ein Screening von cDNA-Banken, die aus Gewebe hergestellt wurden, die das Polypeptid exprimieren, oder von genomischen DNA-Banken mit einer nachweisbar markierten spezifischen Sonde erhältlich. Die Identifizierung positiver cDNA-/genomischer DNA-Klone erfolgt gemäß Standardverfahren; vgl. Maniatis et al., Molecular Cloning (1989), Cold Spring Harbor Laboratory Press. Die Nukleinsäuresequenz, die für das Oligopeptid kodiert, das die erfindungsgemäße Aminosäuresequenz enthält, kann beispielsweise durch Kassetten-mutagenese oder durch rekombinante Polymerasekettenreaktion (PCR) in die doppelsträngige DNA eingefügt werden. Die entsprechenden Methoden sind dem Fachmann bekannt (z.B. Wang et al., 1997; Ruppert et al., 1996). Die erhaltene mutierte doppelsträngige DNA wird sodann in einen Expressionsvektor eingefügt.

Die verwendbaren Vektoren und Wirtszellen wurden bereits vorstehend beschrieben. Wird die Expression in *E. coli* durchgeführt, so kann das Protein aus dem unlöslichen Anteil der Zellen durch Guanidiniumhydrochlorid herausgelöst werden (Ruppert et al., 1996). Sodann wird die Polypeptidvariante renaturiert und über Chromatographie gereinigt. Für die Reinigung von Proteinen aus der TGF-β Superfamilie ist ein pH-Wert von 8 bevorzugt, wobei bevorzugt bei hoher Salzkonzentration (1 M NaCl), in Gegenwart von schwachen Detergentien und mit Redoxmediatoren gearbeitet wird.

Alternativ kann die Polypeptidvariante aus dem Kulturmedium erhalten werden, wenn ein geeigneter Expressionsvektor verwendet wird, der zur Expression der Polypeptidvariante mit einer geeigneten sekretorischen Signalsequenz führt.

Die Erfindung stellt weiterhin pharmazeutische Zusammensetzungen bereit, die mindestens eine erfindungsgemäße Polypeptidvariante und physiologisch verträgliche Zusatzstoffe, die im Stand der Technik bekannt sind, enthalten. Bevorzugt ist hierbei, daß die Polypeptidvariante von einem biologisch aktiven Polypeptid abgeleitet ist, beispielsweise von einem Cytokin oder einem Wachstumsfaktor. Besonders bevorzugt sind hierbei Hormone, Cytokine und Wachstumsfaktoren, die an der Osteogenese beteiligt sind. Die erhöhte Heparin-Bindungsfähigkeit der Polypeptidvarianten führte hierbei zu einer verringerten Diffusion der therapeutisch wirksamen Polypeptidvarianten von der Heparin-Komponente der demineralisierten Knochenmatrix und somit zu einer erhöhten lokalen Konzentration therapeutisch wirksamer Stoffe.

Allgemein wurde auch eine erhöhte Bindungsfähigkeit für extrazelluläre Matrix-Strukturen und Zelloberflächen beobachtet. Die therapeutisch wirksamen Polypeptidvarianten sind somit verwendbar zur Vorbeugung und/oder Behandlung von Krankheiten, an denen die extrazelluläre Matrix oder Zelloberflächen beteiligt oder benachbart sind.

Gehören die den Polypeptidvarianten zugrundeliegenden Polypeptide zur DVR-Familie, so sind sie zur Förderung des Knochenwachstums und -reparatur geeignet. Die Beteiligung von GDF-5 an der Knorpel-Bildung ist beschrieben und für die Sehnen-Entwicklung wird sie postuliert. Somit sind von GDF-5 abgeleiteten Polypeptidvarianten zur Knorpel- und Sehnen-Reparatur geeignet. BMP-7/OP-1 unterdrückt die Produktion von GDF-5, welches möglicherweise einen Mechanismus zum Ausgleich der Knochen- und Knorpel-Bildung darstellt. Somit weisen von BMP-7 abgeleiteten Polypeptidvarianten regulatorische Eigenschaften in bezug auf die Knochen- und Knorpel-Bildung auf.

BMP-7 ist an der Nieren- und Augen-Entwicklung, BMP-6 an der Haut- und BMP-2 an der Herz-Entwicklung beteiligt. Somit können gemäß der vorliegenden Erfindung die von BMP abgeleiteten Polypeptidvarianten zur Regulation der Nieren-, Augen-, Haut- und Herz-Entwicklung verwendet werden.

Weiterhin wird die Angiogenese durch GDF-5 induziert. Daher sind GDF-5 von abgeleiteten Polypeptidvarianten sinnvoll zur Stimulierung der Angiogenese einsetzbar.

Gehören die Polypeptide der Polypeptidvarianten zur TGF-β-Familie, so sind die therapeutisch wirksamen Polypeptidvarianten zur Immunsuppression, Entzündungshemmung, Stimulierung der Knochen- und Knorpel-Bildung geeignet. Sie sind aufgrund der verstärkten Ablagerung von Extrazellulärmatrix-Komponenten wie Collagenen, Fibronectin, Glykosaminoglykanen und Proteoglykanen zur Wundheilung verwendbar.

Weiterhin sind die Polypeptidvarianten auf TGF-β-Basis zur Verhinderung der Retina-Ablösung verwendbar. Ferner sind sie vorteilhaft anwendbar bei oraler Mukositis, die als Nebenwirkung der Tumor-Chemotherapie auftritt. Aufgrund der allgemeinen Zellwachstumshemmung durch TGF-β's können sie zur Unterdrückung von Krebszellen wie beispielsweise Brustkrebszellen verwendet werden.

Die Beteiligung der Hypophysen-Hormone, Aktivine und Inhibine bei der Modulation des weiblichen Menstruationszyklus ist bekannt. Aktivin induziert und Inhibin hemmt die Synthese der Hypophysen-Hormone FSH und LH. Somit wird die Verwendung der erfindungsgemäßen Polypeptidvarianten zur Regulation des weiblichen Menstruationszyklus vorgeschlagen.

Inhibine unterdrücken die Entwicklung von Keimdrüsen-Tumoren. Inhibin-abgeleitete Polypeptidvarianten könnten daher bei der Prophylaxe und zur Behandlung von Keimdrüsen-Tumoren eingesetzt werden.

Aktivine sind vermutlich an der Wundheilung beteiligt. Die Erfindung umfaßt somit die Verwendung von von Aktivin abgeleiteten Polypeptidvarianten zur Herstellung von Medikamenten zur Wundheilung.

Aktivine sind möglicherweise bei der Entwicklung und dem Umsatz von Knorpel zu Knochen beteiligt. Die von Aktivin abgeleiteten Polypeptidvarianten sind somit zur Regulation des Knochen- bzw. Knorpel-Wachstums geeignet.

Aktivin fördert die Expansion von Blast-bildenden Untereinheiten und Kolonie-bildenden Untereinheiten während der Hämatopoese, wohingegen Inhibin diese Funktionen inhibiert. Daher wird die Verwendung von Polypeptidvarianten, die von diesen Proteinen abgeleitet sind, zur Regulation der Hämatopoese vorgeschlagen.

Weitere besonders relevante Polypeptide, die als Grundlage für die Polypeptidvarianten dienen können, sind MIS und GDNF (Glial cell line derived neurotrophic factor), die beide an der Embryonal-Entwicklung beteiligt sind.

In einer weiteren Ausführungsform stellt die Erfindung ferner eine Matrix zur Osteoinduktion bereit, die mindestens eine Polypeptidvariante und einen Träger umfaßt. Weiterhin wird ein *in vitro*-Verfahren zur Osteoinduktion, in dem die osteoinduktive Matrix verwendet wird, vorgeschlagen.

Im Stand der Technik sind osteoinduktive Matrizen vorgeschlagen worden, die Wachstumsfaktoren enthalten. Beispielsweise befindet sich eine von der Firma Integra Life Sciences Corp. hergestellte Collagen-Matrix, die rekombinantes humanes BMP-2 umfaßt, in der Entwicklung (Hollinger et al., 1998). Weiterhin entwickelt die Firma Sofamor - Danek Titankäfige, die rekombinantes humanes BMP-2 enthalten. Schließlich gibt es eine Vorrichtung unter der Bezeichnung "NOVOS™" von der Firma Creative Biomolecules, nun als Weiterentwicklung durch die Firma Stryker bekannt, die osteoinduktives Typ I Knochencollagen und rekombinantes OP-1 (BMP-7) enthält. Diese Vorrichtung befindet sich in der klinischen Phase III in den Vereinigten Staaten von Amerika. Die Verwendungen umfassen die Behandlung von orthopädischen Traumata, maxillofaziale Reparaturen und avaskuläre Nekrosen. Weiterhin wird die Verwendung von BMP-7 zur Behandlung von Knorpelschäden, Nierenversagen, Hirn- und Rückenmarksschädigungen, Herzinfarkt und Osteoporose vorgeschlagen.

Typischerweise wird also ein Matrix-Material mit osteoinduktiven Wachstumsfaktoren versetzt und diese Matrix operativ eingesetzt. Die Matrix ist hierbei nicht nur der Träger des Wachstumsfaktors, sondern verleiht auch physikalische Stabilität und verhindert das Eindringen von Weichteilgewebe in den Ort des Knochendefektes. Das Hinzufügen der Wachstumsfaktoren soll hierbei das Ersetzen des Implantates durch neuen Knochen beschleunigen.

Ein Hauptproblem bei den bekannten Matrix-Typen besteht jedoch darin, daß die in der Matrix enthaltenen Proteine sehr rasch den Ort der Anwendung verlassen. Da die Knochenreparatur jedoch ein relativ langsamer Vorgang auch bei Anwesenheit von Wachstumsfaktoren ist, wirkt die kurze Verbleibzeit des hinzugefügten Proteins limitierend.

Da die biologische Wirkung von osteoinduktiven Wachstumsfaktoren darin besteht, die Immigration von pluripotenten mesenchymalen Zellen unter Bildung von Chondro- und Osteovorläuferzellen zu induzieren und deren Aktivität zu stimulieren, erfordert dies die Lokalisierung der Wachstumsfaktoren an der Reparaturstelle. Wachstumsfaktoren, die aus der Reparaturstelle herausdiffundieren, sind einerseits für die beabsichtigte therapeutische Wirkung sinnlos und bergen andererseits die Gefahr einer ektopischen Knochenbildung.

Die erfindungsgemäßen Polypeptidvarianten zeichnen sich im Vergleich mit den bekannten Polypeptiden durch eine erhöhte Heparin-Bindungsfähigkeit aus, die bei Verwendung von Heparin bzw. Heparin-ähnlichen Strukturen als Matrixmaterial oder auf die Matrix aufgebrachtes Material das rasche Wegdiffundieren der Polypeptidvarianten vom Träger verhindert. Die Polypeptidvarianten bleiben somit am Ort der Wirkung lokalisiert. In einer bevorzugten Ausführungsform besteht der Träger aus Heparin, Hydroxylapatit, Hyaluronsäure, synthetischen Polymeren oder Collagen. Die Trägerstoffe können resorbierbar oder nicht resorbierbar sein.

Im Stand der Technik sind verschiedene Verfahren zum Herstellen von pharmazeutisch verträglichen Matrizen bekannt. Beispiele pharmazeutisch verträglicher Matrizen sind in Wagner et al., 1996, und Fischgrund et al., 1997 beschrieben. Die Matrix kann in Form eines Blockes, Gels, Vlies, einer Kugel oder Granula ausgebildet sein.

Die Verteilung der Polypeptidvarianten Innerhalb der Matrix kann homogen oder nichthomogen sein, wobei eine homogene Verteilung bevorzugt ist Die Verteilung der Polypeptidvariante kann, abhängig von der Größe des Defektes oder der Dauer des Heilungsvorgangs, vorteilhaft gestaltet werden. Die Polypeptidvariante sollte hierbei in einer Konzentration von ungefähr 100 µg/cm³ bis ungefähr 2 mg/cm³, vorzugsweise 250 µg/cm³ bis 750 µg/cm³ und besonders bevorzugt 450 µg/cm³ bis 550 µg/cm³ Träger liegen. In der Regel wird eine Konzentration von ungefähr 500 µg/cm³ verwendet werden.

Erfindungsgemäß kann die osteolnduktive Matrix zur Behandlung von orthopädischen Traumata, maxillofaszlalen Reparaturen und avaskulären Nekrosen verwendet werden. Weiterhin wird die Verwendung zur Prophylaxe und Therapie von Knorpelschäden, Nierenversagen, Hirn- und Rückenmarksschädigungen, Herzinfarkt und Osteoporose vorgeschlagen.

Gegenstand der Erfindung ist die Verwendung einer Polypeptidvariante gemäss der Erfindung, zur Herstellung einer pharmazeutischen Zusammensetzung zur Stimulation der Osteogenese oder Wundheilung, oder zur Behandlung von Entzündung oder Krebs, wobei das as BMP-2, BMP-4, BMP-5, BMP-6, BMP-7/OP-1 und BMP-8/OP-2 ausgewählte Polpeptid durch Addition, Substitution, Insertion, Inversion und/oder Deletion verändert ist, wobei das durch Addition, Substitution, Insertion, Inversion und/oder Deletion veränderte Polpeptid im wesentlichen die gleiche Rezeptorbindungsaffinität zu der Ektodomäne von BNPR-IA wie BMP-2 aufweist und mindestens 50% homolog dazu ist und wobei die Variante die Sequenz der SEQ ID NR: 3 oder 4 umfasst. Bevorzugt ist das obige Polypeptid mindestens 90% homolog zu BMP-2.

Die erfindungsgemäße osteoinduktive Matrix weist bei Verwendung rekombinant hergetellter Polypeptidvarianten weiterhin den Vorteil auf, daß sie frei von Kontaminationen, z. B. Viren ist, die bei Wachstumsfaktoren tierischer Herkunft von Zeit zu Zeit beobachtet werden.

Die erfindungsgemäße osteoinduktive Matrix kann *in vitro* verwendet werden, um in Gewebekultur die Ansiedlung von Osteoblasten und/oder Chondroblasten zu ermöglichen. Diese so vorbereiteten osteoinduktiven Matrizen können sodann autolog oder heterolog mit chirurgischen Verfahren in Patienten eingebracht werden (Kübler et al., 1997; Kübler et al., 1998; Chen et al., 1998).

Es ist beabsichtigt, mit den nachfolgenden Figuren und Beispielen die Erfindung zu erläutern, diese jedoch in keiner Weise einzuschränken. Dem Fachmann sind aufgrund der Beschreibung und der Beispiele weitere Ausführungsformen zugänglich, die ebenfalls umfaßt sind.

### Beschreibung der Figuren:

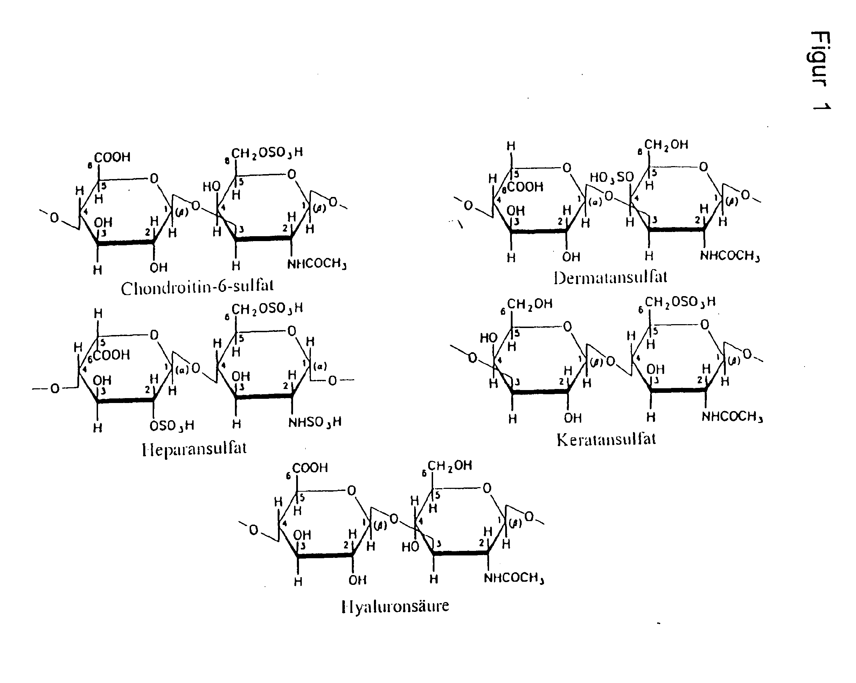

Fig. 1 zeigt Abbildungen einiger Strukturformeln von typischen Disaccharideinheiten Heparin-ähnlicher Strukturen, wie sie unter anderem in bestimmten Glykosaminoglykanen auftreten.

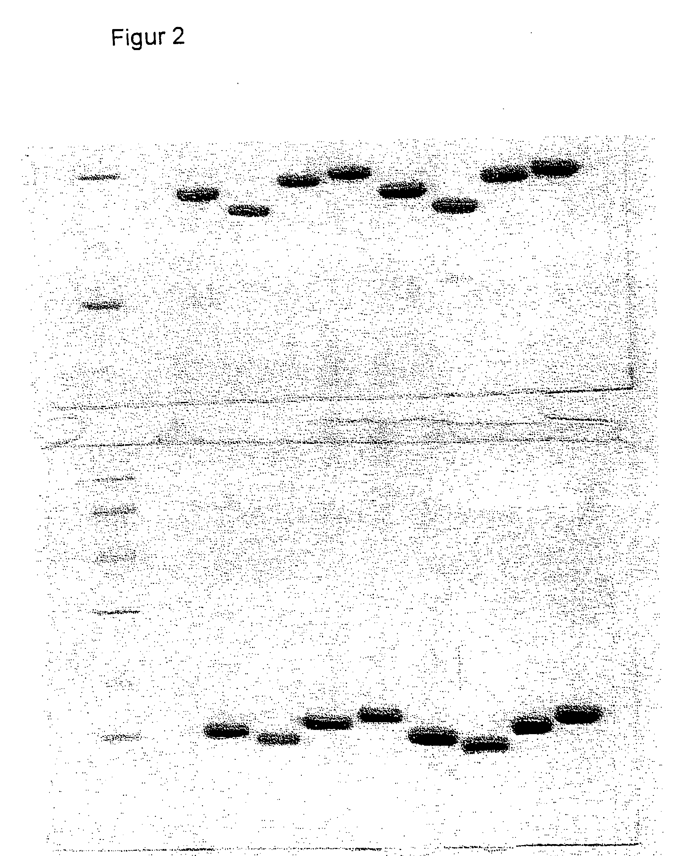

Fig. 2 zeigt das Bild eines mit Coomassie Blau angefärbten SDS-Polyacrylamidgels nach Auftrennung der in *E. coli* exprimierten und gereinigten Varianten T3 und T4 sowie von BMP-2 und EHBMP-2 in oxidierter (oben) und reduzierter (unten) Form. Links ist jeweils ein Molekulargewichtsstandard (15, 20, 30, 35, 68 und 94 kD) aufgetragen. Die Gele wurden (von links nach rechts) wie folgt beladen: Spuren 1-4: je 2 µg BMP-2,
EHBMP-2, T3 (SEQ ID No: 5) und T4 (SEQ ID No: 6); Spuren 5-8: je 5 µg BMP-2, EHBMP-2, T3, T4.

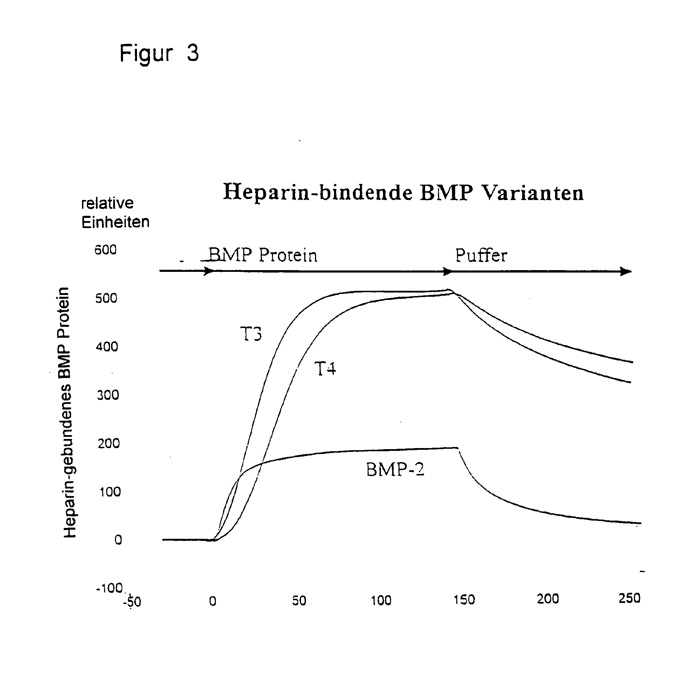

Fig. 3 zeigt eine graphische Darstellung von mit dem Pharmacia BIA2000 System aufgenommenen Sensogrammen der Bindung der Varianten T3 und T4 (SEQ ID No: 5 und 6) sowie von BMP-2 an Heparin.

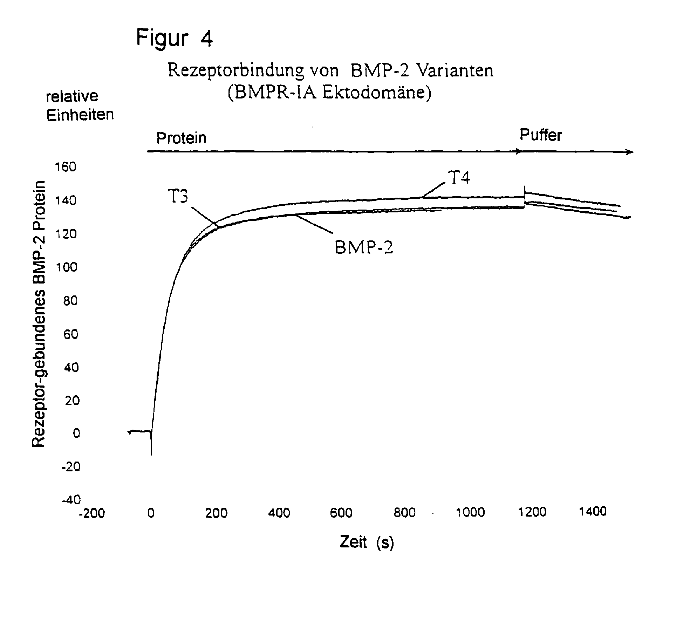

Fig. 4 zeigt eine graphische Darstellung von mit dem BIA2000 System aufgenommenen Sensogrammen der Bindung der Varianten T3 und T4 (SEQ ID No: 5 und 6) sowie von BMP-2 an die Ektodomäne des Rezeptors BMPR-IA.

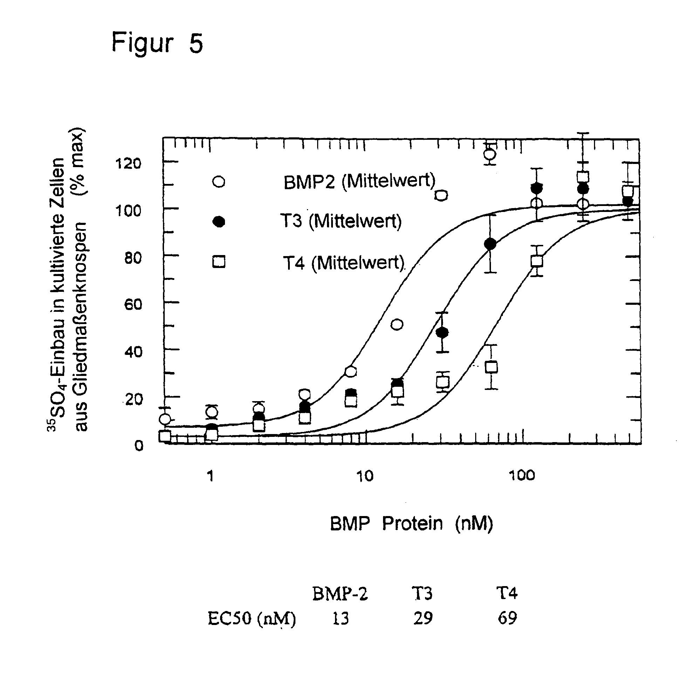

Fig. 5 zeigt in einer graphischen Darstellung den Einbau von ³⁵SO₄ in kultivierte Zellen aus Gliedmaßenknospen von Hühnerembryonen in Abhängigkeit von der Konzentration von BMP-2, der Variante T3 und der Variante T4 (SEQ ID No. 5 und 6).

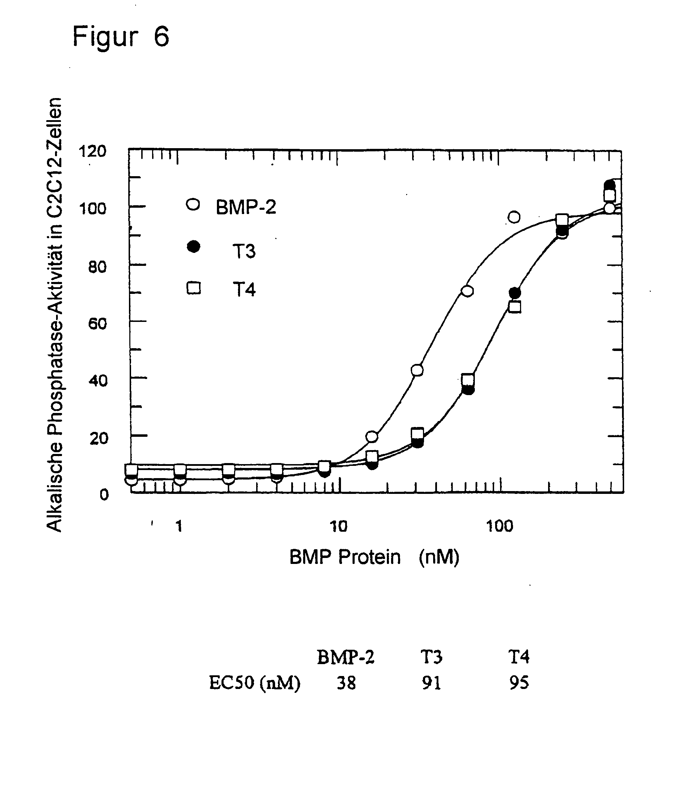

Fig. 6 zeigt in einer graphischen Darstellung die Induktion der Alkalischen Phosphatase in kultivierten C2C12 Zellen in Abhängigkeit von der Konzentration von BMP-2, der Variante T3 und der Variante T4 (SEQ ID No. 5 und 6).
Fig. 7a und 7b zeigen histologische Ansichten von in der Maus gebildeten Ossikeln, die durch Implantation von BMP-2 induziert wurden. Die Präparate wurden mit Hematoxylin-Eosin gefärbt. Ossikel erscheinen violett, mit darin eingeschlossenen weissen oder dunkelvioletten Arealen. Bei den dunkelvioletten Bereichen handelt es sich um Knochenmark, bei den weissen Zellen um Fettgewebe, das natürlicherweise im Knochenmark vorkommt. Die umgebende Muskulatur ist intensiv rot gefärbt.
Fig. 8 zeigt die histologische Ansicht eines in der Maus gebildeten Ossikels, das durch Behandlung mit T3 induziert wurde. Das Präparat wurde ebenfalls mit Hematoxylin-Eosin gefärbt.
Fig. 9 zeigt eine Röntgenaufnahme eines durch T3-lmplantation in der Maus gebildeten Ossikels.
Fig. 10 zeigt die histologische Ansicht eines in der Maus gebildeten Ossikels, das durch Behandlung mit T3 induziert wurde.

### Methoden

### Messung der Heparin-Bindung

Zur Messung der Heparin-Bindung wurde Heparin 6000 aminobiotinyliert (Mach et al., 1993) und an einen Streptavidin-beschichteten Biosensor CM5 (Pharmacia Biosensor AB) fixiert. Die Bindung von Polypeptiden und erfindungsgemäßen Polypeptidvarianten mit erhöhter Heparin-Bindungsfähigkeit an den Heparin-dotierten Biosensor wurde mit Hilfe eines BIA2000 Gerätes gemessen. Die einzelnen Versuchsbedingungen sind vorbeschrieben (Ruppert et al., 1996).

### Messung der Bindung an den Rezeptor BMPR-IA

Zur Messung der Bindung wurde die Ektodomäne des Rezeptors BMPR-IA aminobiotinyliert (Shen et al., 1996) und an einen Streptavidin-beschichteten Biosensor CM5 (Pharmacia Biosensor AB) fixiert. Die Bindung von Polypeptiden und den erfindungsgemäßen Polypeptidvarianten an den Rezeptor-dotierten Biosensor wurde mit Hilfe eines BIA2000 Gerätes gemessen.

### Messung der biologischen Osteoinduktions-Aktivität

Zur Messung der biologischen Aktivität wurde folgendes Zellkultursystem verwendet: Zellen aus den Gliedmaßenknospen von Hühnerembryonen wurden isoliert und für die Messung des BMP-abhängigen Einbaus von ³⁵SO₄ in Proteoglykane verwendet (Ruppert et al., 1996). Es wurde ein Konzentrationsbereich für das BMP-Protein gewählt, der es ermöglichte, sowohl die maximale Zellantwort als auch die EC₅₀-Konzentration (Konzentration, bei der 50 % des maximalen Einbaus erreicht werden) zu bestimmen.

Die myoblastäre Maus-Zellinie C2C12 wurde verwendet, um die BMP-abhängige Induktion der Alkalischen Phosphatase zu bestimmen (Katagiri et al., 1994). Es konnten die maximale Zellantwort und die EC₅₀-Konzentration bestimmt werden.

### Beispiel 1: Expression und Charakterisierung von T3

Es wurde cDNA (Ruppert et al., 1996), die für das reife humane BMP-2 (NIH-Datenbank Entrez/Swiss-Prot Nr. P12643) und zusätzlich ATGGCT (Met-Ala) am 5' Ende kodierte, einer Kassetten-Mutagenese (Wang et al., 1997) unterworfen. Zwischen die singulären Spaltstellen für *Nco*I und *Afl*II wurde folgende doppelsträngige DNA eingefügt:
5' CATGGCTCAAGCCAAACACAAACAGCGGAAACGCGCTCGTAAACGTC 3' SEQ ID NO: 9
3' CGAGTTCGGTTTGTGTTTGTCGCCTrPGCGCGCGCATTTGCAGAATT 5 SEQ ID NO: 10

Dadurch wurde zwischen Gln an Position 8 und Arg an Position 9 eines humanen Met-Ala-BMP-2 zusätzlich die Sequenz Arg-Lys-Arg-Ala (SEQ ID No. 3) eingefügt. Die mutierte cDNA wurde als *Nco*I/*Bam*HI-Fragment in den Expressionsvektor pRBSIIP_{N25}x/o (Stueber et al., 1984) integriert, und die Mutation durch Sequenzierung bestätigt.

Nach Expression und Isolierung zeigte sich, daß die so hergestellte Variante T3 in der SDS-Polyacrylamid-Gelelektrophorese (SDS-PAGE) ein im Vergleich zu BMP-2 erwartungsgemäß höheres apparentes Molekulargewicht aufwies. T3 interagierte in Biosensor Experimenten (BIA2000) mit unveränderter Affinität (Dissoziationskonstante K_{d}: ca. 200 pM) mit der Ektodomäne des BMP-Rezeptors BMPR-IA (NIH-Datenbank Entrez/Swiss-Prot Nr. P36894). Die Bindung an Heparin war jedoch im Vergleich zu BMP-2 erhöht, wohingegen die Abdissoziierung verlangsamt war.

Die biologische Aktivität war in verschiedenen Testsystemen verändert. Die über einen Sulfateinbau gemessene Proteoglykansynthese in Zellen der Gliedmaßenknospen von Hühnerembryonen zeigte einen höheren EC₅₀-Wert für T3 im Vergleich zu BMP-2. Der maximal erreichbare Einbau war unverändert. Die Induktion der Alkalischen Phosphatase-Aktivität in der C2C12-Zellinie zeigte für T3 ebenfalls einen höheren EC₅₀-Wert als für BMP-2.

### Beispiel 2: Expression und Charakterisierung von T4

Es wurde Beispiel 1 mit der Ausnahme wiederholt, daß folgende doppelsträngige DNA in die BMP-2 cDNA integriert wurde:

Die obere Sequenz wird mit SEQ ID NO: 11, die untere mit SEQ ID NO: 12 im Sequenzprotokoll angegeben.

Dadurch wurde zwischen Gin an Position 8 und Arg an Position 9 des BMP-2 zusätzlich die Sequenz Arg-Lys-Arg-Ala-Lys-His-Lys-Gln eingefügt.

Die so exprimierte und isolierte Variante T4 wies in der SDS-PAGE ein höheres apparentes Molekulargewicht als T3 und BMP-2 auf. T4 bindet an die Ektodomäne des BMP-Rezeptors BMPR-IA mit einer Dissoziationskonstante von etwa 340 pM. Dies entspricht der Rezeptor-Affinität von BMP-2 (K_{d} 320 pM). Die Bindung von T4 an Heparin war jedoch im Vergleich zu BMP-2 erhöht, und die Abdissoziierung verlangsamt. Die Freisetzung von T4 von Heparin war überraschenderweise langsamer als die von T3.

Die biologische Aktivität von T4 ist im Vergleich zu BMP-2 verändert. T4 zeigt während der Proteoglykansynthese (Sulfateinbau) in Zellen von Gliedmaßenknospen von Hühnerembryonen einen höheren EC₅₀-Wert als BMP-2 (und sogar höher als T3). Ebenso wird die Aktivität der Alkalischen Phosphatase in C2C12-Zellen durch T4 bei einem höheren EC₅₀-Wert induziert als durch BMP-2.

### Beispiel 3: Untersuchungen zur in vivo-Wirksamkeit der Polypeptidvarianten

Als Methode wurde hierbei die Induktion von ektotopem Knochen im Oberschenkelmuskel der Maus gewählt. Bei ektoper Knochenbildung entsteht Knochen *de novo* in einem fremden Gewebe und ohne Kontakt zum Skelettsystem. Der Vorteil dieses Testsystems liegt in seiner hohen Stringenz. Da kein Kontakt mit skelettalem Knochen existiert, spielen regenerative Knochenheilungsprozesse keine Rolle. Somit werden falsch positive Versuchsergebnisse vermieden, die durch Verletzung des Knochens während der Operation verursacht werden könnten.

Die Methode zur Induktion ektoper Knochenbildung in ICR-Mäusen ist im Detail beschrieben in Kübler und Urist, 1991.

BMP-2 und die Variante T3 wurden in unterschiedlichen Konzentrationen mit Rinderserumalbumin als Träger vermischt, und in den Musculus quadriceps femoris von ICR-Mäusen implantiert. Nach 3 Wochen wurde das gebildete Knochenmaterial durch Röntgenaufnahmen und histologische Untersuchungen charakterisiert.

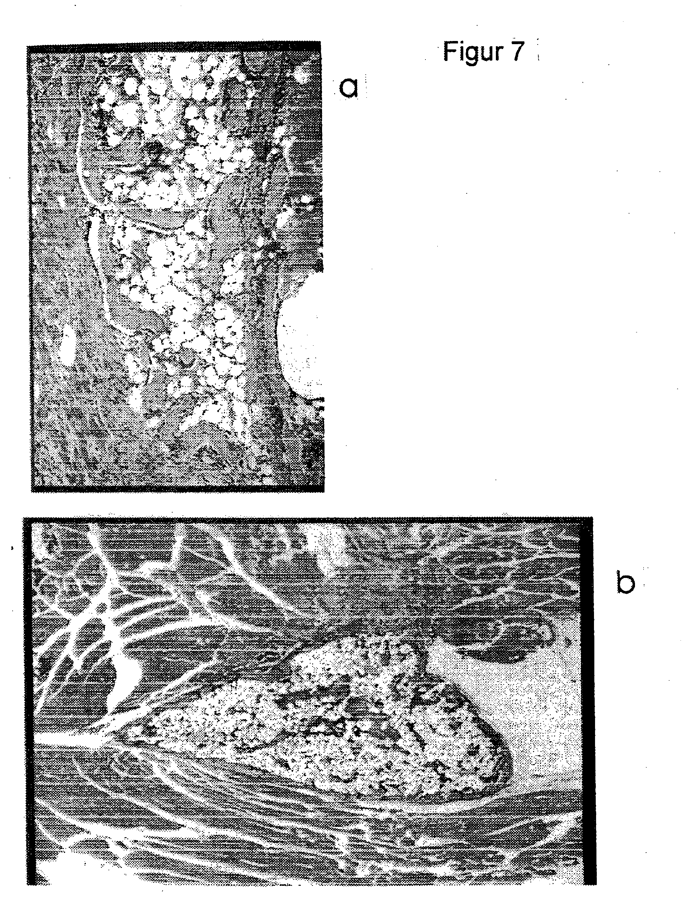

Die Implantation von Rinderserumalbumin ohne BMP (Kontrolle) ergab in keinem Fall nachweisbare Knochenbildung. Weder mit BMP-2 noch mit T3 wurden Anzeichen für eine Entzündung oder sonstige schädliche Nebenwirkungen beobachtet. Fig. 7 ist zu entnehmen, daß BMP-2 die Bildung von Knochenmatrix und Knochenmark induzieren konnte. Fig. 8 zeigt die entsprechende Abbildung für die Behandlung mit T3. Im Vergleich zum Ergebnis mit BMP-2 zeigt sich für T3, daß das Verhältnis von Knochenmatrix zu Knochenmark stark in Richtung eines hohen Anteils von Knochenmatrix verschoben ist.

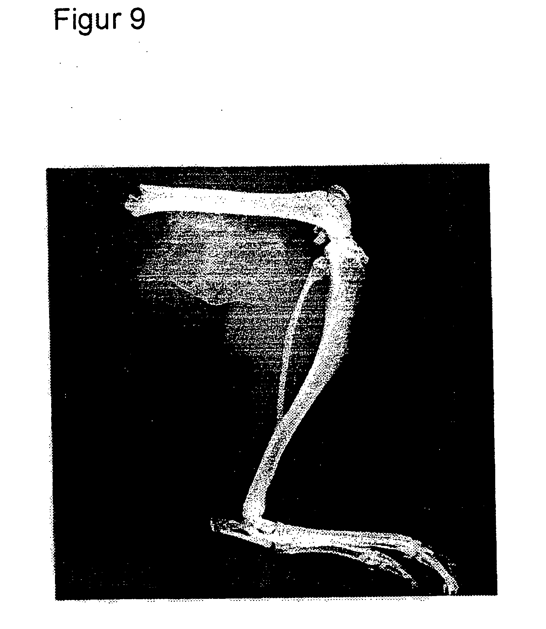

Fig. 9 zeigt eine Röntgenaufnahme eines durch T3 Implantation gebildeten Ossikels. Die Größe des Ossikels unterscheidet sich nicht wesentlich von Ossikeln, die im gleichen Testsystem mit konventionellen BMPs erhalten wurden.

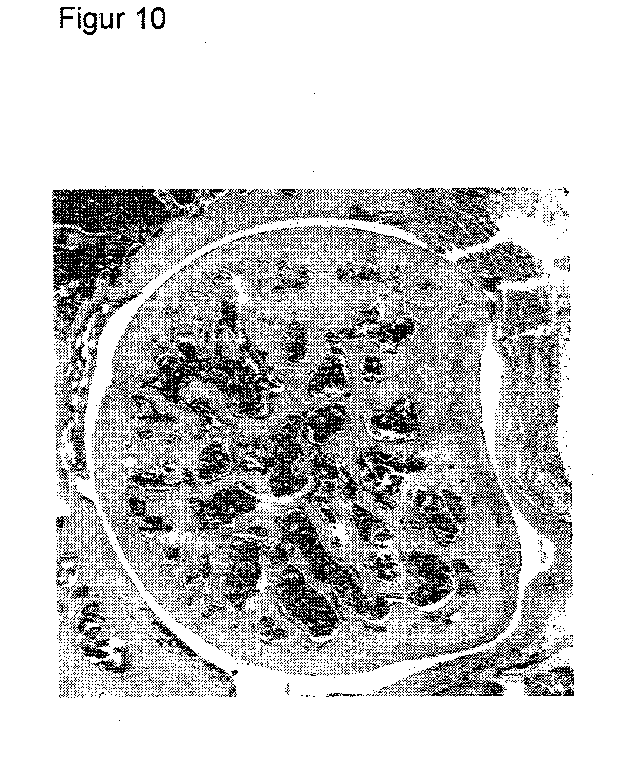

Fig. 10 zeigt die histologische Ansicht eines Ossikels, das durch Behandlung mit T3 induziert wurde. Die Färbung wurde hier mit Masson-Trichrome durchgeführt, was es erlaubt, den Differenzierungsstatus von Knochengewebe zu unterscheiden. Rote Farbe zeigt komplett ausdifferenzierten Knochen an, türkise Areale sind noch im Prozess der Ossifikation begriffen. Zum Teil können in dem türkis gefärbten Gewebe runde, weisse Zellen ausgemacht werden; es handelt sich dabei um Restanteile von knorpelbildenden Chondrocyten, da die BMP-induzierte Knochenbildung durch endochondrale Ossifikation erfolgt (transiente Knorpelbildung).

Die den Figuren 7 bis 10 zugrundeliegenden Versuche werden mit jeweils 10 µg rekombinantem humanem BMP-2 bzw. rekombinanten T3 (SEQ ID No. 5) durchgeführt. In der folgenden Tabelle wird die *in vivo* Wirksamkeit von BMP-2 und rekombinantem T3 verglichen.

**Tabelle 1: Vergleich der durch BMP-2 bzw. T3-induzierten Knochenbildung.**

| **rhBMP-2 (**µ**g)** | **T3 (**µ**g)** | **Knochenbildung** |
|---|---|---|
| - | - | 0/30 |
| 0,4 | - | 0/3 |
| - | 0,4 | 3/4 |
| 1 | - | 0/9 |
| - | 1 | 4/4 |
| 4 | - | 3/3 |
| - | 4 | 4/4 |
| 10 | - | 10/11 |
| - | 10 | 4/4 |

Es zeigt sich, daß T3 bei geringen Konzentrationen wirksamer ist als BMP-2 (Tabelle 1). Bei Implantation von 1 µg BMP-2 erfolgte in keinem von neun Versuchen Knochenbildung, wohingegen bei der gleichen Menge T3 in vier von vier implantierten Tieren Knochen gebildet wurde. T3 induziert sogar bei Applikation von nur 0,4 µg noch in 3 von 4 Tieren Knochenbildung.

Die Ergebnisse zeigen, daß die Polypeptidvariante mit erhöhter Heparin-Bindungsfähigkeit *in vivo* die Knochenbildung induzieren kann. Es wurden in keinem Fall Entzündungsreaktionen oder sonstige Unverträglichkeiten beobachtet. Im Vergleich zu konventionellem BMP-2 bewirkt die Anwendung von T3 die Bildung eines Ossikels mit wesentlich höherem Anteil an Knochenmatrix. Da die Matrix dem Knochen seine mechanische Stabilität verleiht, wird ein in diesem Sinne dichterer Knochen eine wesentlich höhere Festigkeit und funktionelle Belastbarkeit aufweisen. Es wird also im Vergleich zu BMP-2 die Bildung eines Knochens mit deutlich höherer Qualität induziert. Da T3 bei einer geringeren Konzentration als BMP-2 *in vivo* biologische Wirksamkeit zeigt, kann auch die erforderliche Menge an Wachstumsfaktor reduziert werden. Dies stellt einen wünschenswerten Vorteil der Polypeptidvariante im Vergleich zu BMP-2 dar.

### Literaturliste

Altschul et al., J. Mol. Biol. 215 (1990), 403-410
Altschul et al., BLAST Handbuch, NCB NLM NIH Bethesda MD 20894
Chen et al., Neurosurg. Focus 4 (1998), Artikel 11
Devereux et al., Nucleic Acid Res. 12 (1984), 387
Fischgrund et al., J. Spinal Disorder 10 (1997), 467-472
Henikoff & Henikoff, PNAS USA 89 (1992), 10915-10919
Hollinger et al., J. Biomed. Mater. Res. 43 (1998), 356-364
Katagiri et al., J. Cell. Biol. 127 (1994), 1755-66; Erratum publiziert in J. Cell. Biol. 128 (1995), 714
Kingsley, Genes Dev. 8 (1994), 133-146
Kübler et al., Dtsch. Zahnärztl. Z. 53 (1998), 834-843
Kübler et al., Mund-, Kiefer-, Gesichtschirurgie 1 (1997), 2-25
Kübler et al., Mund-, Kiefer-, Gesichtschirurgie 3 (Suppl. 1) (1999), 134-139
Kübler & Urist, J. Craniomaxillofac. Surg. 19 (1991), 283-288
Mach et al., Biochemistry 32, (1993), 5480-89
Maniatis et al., Molecular Cloning (1989), Cold Spring Harbor Laboratory Press McDonald & Hendrickson, Cell 73 (1993), 421-24
Needleman & Wunsch, J. Mol. Biol. 48 (1970), 443-453
Recombinant Gene Expression Protocols, Hrsg. R. S. Tuan Methods in Molecular Biology 62 (1995), Humana Press, Totowa, New Jersey
Reddi, Nature Biotechnol. 16 (1998), 247-52
Ruppert et al., Eur. J. Biochem. 237 (1996), 295-302
Shen et al., Eur. J. Biochem. 240 (1996), 252-261
Stueber et al., EMBO J. 3 (1984), 3143-3148
Wagner et al., J. Surg. Res. 66 (1996), 100-108
Wang et al., PNAS 94 (1997), 1657-62
Weigel et al., Eur. J. Biochem. 180 (1989), 295-300

## Patentansprüche

1. Polypeptidvariante eines Polypeptids, das aus Mitgliedern der TGFβ-Superfamilie ausgewählt ist, **dadurch gekennzeichnet, dass** die Polypeptidvariante eine erhöhte Heparinbindungsfähigkeit aufweist, wobei an die Aminosäuresequenz des Polypeptids, ausgewählt aus Mitgliedern der TGFβ-Superfamilie, wenigstens ein Oligopeptid addiert und/oder inseriert ist und/oder wenigstens eine natürlicherweise in der Aminosäuresequenz des Polypeptids vorkommende Oligopeptidsequenz durch ein Oligopeptid substituiert ist, wobei das Oligopeptid die Aminosäuresequenz RKRA (SEQ ID NO: 3) oder RKRAKHKQ (SEQ ID NO: 4) umfaßt.

2. Polypeptidvariante gemäß Anspruch 1, **dadurch gekennzeichnet, dass** ein bis vier Kopien des Oligopeptides an ein bis vier Positionen in das Polypeptid eingefügt sind.

3. Polypeptidvariante gemäß einem der Ansprüche 1 bis 2, **dadurch gekennzeichnet, dass** das Oligopeptid an den N-Terminus addiert ist und/oder in den N-terminalen Bereich inseriert ist und/oder einen Teil des N-terminalen Bereiches substituiert.

4. Polypeptidvariante gemäß einem der Ansprüche 1 bis 3, **dadurch gekennzeichnet, dass** die Aminosäuresequenz der Polypeptidvariante ferner eine für die rekombinante Expression relevante Sequenz am N-Terminus umfaßt, wobei die für die rekombinante Expression relevante Sequenz M oder MZ ist und wobei M Methionin und Z eine oder mehrere beliebige Aminosäuren bedeutet.

5. Polypeptidvariante gemäß einem der Ansprüche 1 bis 4, **dadurch gekennzeichnet, dass** die Polypeptidvariante ferner ein His-Tag umfaßt.

6. Polypeptidvariante gemäß einem der Ansprüche 1 bis 5, **dadurch gekennzeichnet, daß** das aus BMP-2, BMP-4, BMP-5, BMP-6, BMP-7/OP-1 und BMP-8/OP-2 ausgewählte Polypeptid durch Addition, Substitution, Insertion, Inversion und/oder Deletion verändert ist, wobei das durch Addition, Substitution, Insertion, Inversion und/oder Deletion veränderte Polypeptid im wesentlichen die gleiche Rezeptorbindungsaffinität zu der Ektodomäne von BNPR-IA wie BMP-2 aufweist und mindestens 50% homolog dazu ist und wobei die Variante die Sequenz der SEQ ID Nr: 3 oder 4 umfasst.

7. Polypeptidvariante gemäß einem der Ansprüche 1 bis 6, **dadurch gekennzeichnet, dass** das Polypeptid BMP-2, BMP-4, BMP-5, BMP-6, BMP-7/OP-1 oder BMP-8/OP-2 ist.

8. Polypeptidvariante gemäß einem der Ansprüche 1 bis 7, **dadurch gekennzeichnet, dass** das Oligopeptid vor dem Cystinknoten eingefügt ist.

9. Polypeptidvariante gemäß einem der Ansprüche 7 bis 8, **dadurch gekennzeichnet, dass** die Polypeptidvariante die Aminosäuresequenz SEQ IID NO: 5 (T3) oder SEQ ID NO: 6 (T4) hat.

10. Polypeptidvariante gemäß einem der Ansprüche 1 bis 9, **dadurch gekennzeichnet, dass** die Polypeptidvariante ein Poly-, Oligo- oder Dimer der Polypeptidvariante gemäß einem der Ansprüche 1 bis 9 ist.

11. Nukleinsäuremolekül, umfassend eine für eine Polypeptidvariante gemäß einem der Ansprüche 1 bis 10 kodierende Nukleinsäuresequenz.

12. Nukleinsäuremolekül gemäß Anspruch 11, **dadurch gekennzeichnet, dass** die Nukleinsäuresequenz von einer genomischen DNA oder cDNA abgeleitet oder eine synthetische DNA ist.

13. Nukleinsäuremolekül gemäß einem der Ansprüche 11 oder 12, weiterhin umfassend einen zur Expressionskontrolle geeigneten Promotor, wobei die für eine Polypeptidvariante kodierende Nukleinsäuresequenz unter der Kontrolle des Promotors steht.

14. Nukleinsäuremolekül gemäß einem der Ansprüche 11 bis 13, wobei das Nukleinsäuremolekül wenigstens einen Teil eines Vektors enthält.

15. Wirtszelle, enthaltend ein Nukleinsäuremolekül gemäß einem der Ansprüche 11 bis 14, wobei die Wirtszelle eine zur Expression des Nukleinsäuremoleküls geeignete prokaryontische oder eukaryontische Zelle ist.

16. Verfahren zum Herstellen einer Polypeptidvariante mit erhöhter Heparin-Bindungsfähigkeit gemäß Anspruch 1, umfassend das Addieren wenigstens eines Oligopeptides, das eine Aminosäuresequenz enthält, die ausgewählt ist aus: SEQ ID NO: 3 und SEQ ID NO: 4, an die Aminosäuresequenz eines Polypeptides;
und/oder das Inserieren wenigstens eines Oligopeptides, das eine Aminosäuresequenz enthält, die ausgewählt ist aus: SEQ ID NO: 3 und SEQ ID NO: 4, in die Aminosäuresequenz eines Polypeptides;
und/oder das Substituieren wenigstens einer natürlicherweise in der Aminosäuresequenz eines Polypeptids vorkommenden Oligopeptidsequenz durch ein Oligopeptid, das eine Aminosäuresequenz enthält, die ausgewählt ist aus: SEI ID NO: 3 und SEQ ID NO: 4.

17. Verfahren gemäß Anspruch 16, **dadurch gekennzeichnet, dass** das Verfahren ein chemisches und/oder enzymatisches Syntheseverfahren umfaßt.

18. Verfahren gemäß einem der Ansprüche 16 oder 17, **dadurch gekennzeichnet, dass** das Verfahren gentechnologische Verfahren umfaßt.

19. Verfahren gemäß einem der Ansprüche 16 bis 18, **dadurch gekennzeichnet, dass** das Verfahren umfasst:
*a) in vitro* Mutagenese einer für ein Polypeptid kodierenden Nukleinsäure, so dass (i) an die für das Polypeptid kodierende Nukleinsäure wenigstens eine Nukleinsäure addiert wird, die für ein Oligopeptid kodiert, das eine Aminosäuresequenz, ausgewählt aus SEQ ID Nos. 3 und 4, enthält; und/oder (ii) in die für das Polypeptid kodierende Nukleinsäure wenigstens eine Nukleinsäure inseriert wird, die für ein Oligopeptid kodiert, das eine Aminosäuresequenz, ausgewählt aus SEQ ID Nos. 3 und 4, enthält; und/oder (iii) wenigstens eine natürlicherweise in der für das Polypeptid kodierenden Nukleinsäure vorkommende Nukleinsäuresequenz durch eine Nukleinsäuresequenz substituiert wird, die für ein Oligopeptid kodiert, das eine Aminosäuresequenz, ausgewählt aus: SEQ ID NO: 3 und SEQ ID NO: 4, enthält;
*b)* Einklonieren der mutierten Nukleinsäure in einen geeigneten Expressionsvektor;
*c)* Transformieren/Transfizieren einer geeigneten Wirtszelle mit dem erhaltenen Expressionsvektor;
*d*) Kultivieren der transformierten/transfizierten Wirtszelle unter zur Expression geeigneten Bedingungen;
*e)* Isolierung und gegebenenfalls Renaturieren der exprimierten Polypeptidvariante.

20. Verfahren gemäß einem der Ansprüche 16 bis 19, **dadurch gekennzeichnet, dass** das Verfahren in einer prokaryontischen Wirtszelle, vorzugsweise *E. coli,* durchgeführt wird.

21. Verfahren gemäß einem der Ansprüche 16 bis 19, **dadurch gekennzeichnet, dass** das Verfahren in einer eukaryontischen Zelle, bevorzugt einer Hefe-, Pflanzen-, Insekten-, CHO- oder COS-Zelle, durchgeführt wird.

22. Pharmazeutische Zusammensetzung, umfassend eine Polypeptidvariante gemäß einem der Ansprüche 1 bis 10 und gegebenenfalls physiologisch verträgliche Zusatzstoffe.

23. Verwendung einer Polypeptidvariante gemäß einem der Ansprüche 1 bis 10 zur Herstellung einer pharmazeutischen Zusammensetzung zur Stimulierung der Osteogenese oder Wundheilung, oder zur Behandlung von Entzündung oder Krebs.

24. Zusammensetzung zur Osteoinduktion, umfassend eine Polypeptidvariante gemäß einem der Ansprüche 1 bis 10 und einen Träger, ausgewählt aus Heparin, Hydroxylapatit, Hyaluronsäure, synthetischen Polymeren und Collagen.

25. Osteoinduktive Matrix, **dadurch gekennzeichnet, dass** sie Heparin oder Heparin-ähnliche Substanzen enthält oder damit beschichtet ist und an das Heparin oder die Heparin-ähnlichen Substanzen Polypeptidvarianten gemäß einem der Ansprüche 1 bis 10 adsorbiert sind.

26. Verwendung einer Polypeptidvariante nach einem der Ansprüche 1 bis 5, zur Herstellung einer pharmazeutischen Zusammensetzung zur Stimulation der Osteogenese oder Wundheilung, oder zur Behandlung von Entzündung oder Krebs, wobei das aus BMP-2, BMP-4, BMP-5, BMP-6, BMP-7/OP-1 und BMP-8/OP-2 ausgewählte Polypeptid durch Addition, Substitution, Insertion, Inversion und/oder Deletion verändert ist, wobei das durch Addition, Substitution, Insertion, Inversion und/oder Deletion veränderte Polypeptid im wesentlichen die gleiche Rezeptorbindungsaffinität zu der Ektodomäne von BNPR-IA wie BMP-2 aufweist und mindestens 50% homolog dazu ist und wobei die Variante die Sequenz der SEQ ID Nr: 3 oder 4 umfasst.

27. Verwendung einer Polypeptidvariante nach Anspruch 26, wobei das aus veränderte Polypeptid mindestens 90% homolog zu BMP-2 ist.

## Claims

1. Polypeptide variant of a polypeptide selected from the members of the TGFβ superfamily, **characterised in that** the polypeptide variant exhibits an elevated heparin-binding ability, at least one oligopeptide being added to and/or inserted into the amino acid sequence of the polypeptide selected from the members of the TGFβ family and/or at least one oligopeptide sequence which occurs naturally in the amino acid sequence of the polypeptide being substituted by an oligopeptide, the oligopeptide comprising the amino acid sequence RKRA (SEQ ID NO: 3) or RKRAKHKQ (SEQ ID NO: 4).

2. Polypeptide variant according to claim 1, **characterised in that** one to four copies of the oligopeptide are inserted into the polypeptide at one to four positions.

3. Polypeptide variant according to either claim 1 or claim 2, **characterised in that** the oligopeptide is added to the N-terminus and/or inserted into the N-terminal region, and/or substitutes a part of the N-terminal region.

4. Polypeptide variant according to any one of claims 1 to 3, **characterised in that** the amino acid sequence of the polypeptide variant further comprises a sequence of relevance to recombinant expression at the N-terminus, said sequence of relevance to recombinant expression being M or MZ, where M stands for methionine and Z stands for one or more amino acids.

5. Polypeptide variant according to any one of claims 1 to 4, **characterised in that** the polypeptide variant further contains a His-tag.

6. Polypeptide variant according to any one of claims 1 to 5, **characterised in that** the polypeptide selected from BMP-2, BMP-4, BMP-5, BMP-6, BMP-7/OP-1 and BMP-8/OP-2 is altered by addition, substitution, insertion, inversion, and/or deletion, the polypeptide altered by addition, substitution, insertion, inversion and/or deletion exhibiting essentially the same receptor binding affinity to the ectodomain of BMPR-IA as BMP-2, and being at least 50 % homologous thereto, the variant comprising the sequence of SEQ ID NO: 3 or 4.

7. Polypeptide variant according to any one of claims 1 to 6, **characterised in that** the polypeptide is BMP-2, BMP-4, BMP-5, BMP-6, BMP-7/OP-1 or BMP-8/OP-2.

8. Polypeptide variant according to any one of claims 1 to 7, **characterised in that** the oligopeptide is inserted before the cysteine knot.

9. Polypeptide variant according to either claim 7 or claim 8, **characterised in that** the polypeptide variant has the amino acid sequence SEQ ID NO: 5 (T3) or SEQ ID NO: 6 (T4).

10. Polypeptide variant according to any one of claims 1 to 9, **characterised in that** the polypeptide variant is a polymer, oligomer, or dimer of the polypeptide variant according to any one of claims 1 to 9.

11. Nucleic acid molecule, comprising a nucleic acid sequence encoding a polypeptide variant according to any one of claims 1 to 10.

12. Nucleic acid molecule according to claim 11, **characterised in that** the nucleic acid sequence is derived from genomic DNA or cDNA, or is synthetic DNA.

13. Nucleic acid molecule according to either claim 11 or claim 12, further comprising a promoter suited to control expression, wherein the nucleic acid sequence encoding a polypeptide variant is under the control of the promoter.

14. Nucleic acid molecule according to any one of claims 11 to 13, wherein the nucleic acid molecule contains at least part of a vector.

15. Host cell containing a nucleic acid molecule according to any one of claims 11 to 14, wherein the host cell is a prokaryotic or eukaryotic cell suitable for expression of the nucleic acid molecule.

16. Method of producing a polypeptide variant with elevated heparin-binding ability according to claim 1, comprising: the addition of at least one oligopeptide containing an amino acid sequence selected from SEQ ID NO: 3 and SEQ ID NO: 4 to the amino acid sequence of a polypeptide; and/or the insertion of at least one oligopeptide containing an amino acid sequence selected from SEQ ID NO: 3 and SEQ ID NO: 4 into the amino acid sequence of a polypeptide; and/or substitution of at least one oligopeptide sequence naturally occurring within the amino acid sequence of a polypeptide with an oligopeptide containing an amino acid sequence selected from SEQ ID NO: 3 and SEQ ID NO: 4.

17. Method according to claim 16, **characterised in that** the method comprises a chemical and/or enzymatic synthesis method.

18. Method according to either claim 16 or claim 17, **characterised in that** the method comprises gene technology methods.

19. Method according to any one of claims 16 to 18, **characterised in that** the method comprises:
a) *in vitro* mutagenesis of a nucleic acid encoding a polypeptide, so that (i) at least one nucleic acid encoding an oligopeptide containing an amino acid sequence selected from SEQ ID NOS: 3 and 4 is added to the nucleic acid encoding the polypeptide; and/or (ii) at least one nucleic acid encoding an oligopeptide containing an amino acid sequence selected from SEQ ID NOS: 3 and 4 is inserted into the nucleic acid encoding the polypeptide; and/or (iii) at least one nucleic acid sequence naturally occurring within the nucleic acid sequence encoding the polypeptide is substituted with a nucleic acid sequence encoding an oligopeptide containing an amino acid sequence selected from SEQ ID NOS: 3 and 4;
b) cloning of the mutated nucleic acid into a suitable expression vector;
c) transformation/transfection of a suitable host cell with the expression vector obtained;
d) cultivation of said transformed/transfected host cell under conditions suitable for expression;
e) isolation, and if necessary renaturation, of the expressed polypeptide variant.

20. Method according to any one of claims 16 to 19, **characterised in that** the method is carried out in a prokaryotic host cell, preferably *E. coli*.

21. Method according to any one of claims 16 to 19, **characterised in that** the method is carried out in a eukaryotic cell, preferably a yeast cell, a plant cell, an insect cell, a CHO cell or a COS cell.

22. Pharmaceutical composition comprising a polypeptide variant according to any one of claims 1 to 10 and optionally comprising physiologically acceptable additives.

23. Use of a polypeptide variant according to any one of claims 1 to 10 for the production of a pharmaceutical composition for stimulating osteogenesis or wound healing, or for the treatment of inflammation or cancer.

24. Composition for osteoinduction, comprising a polypeptide variant according to any one of claims 1 to 10 and a carrier selected from heparin, hydroxyapatite, hyaluronic acid, synthetic polymers and collagen.

25. Osteoinductive matrix, **characterised in that** it contains or is coated with heparin or heparin-like substances, and polypeptide variants according to any one of claims 1 to 10 are adsorbed to the heparin or heparin-like substances.

26. Use of a polypeptide variant according to any one of claims 1 to 5, for the production of a pharmaceutical composition for stimulating osteogenesis or wound healing, or for the treatment of inflammation or cancer, wherein the polypeptide selected from BMP-2, BMP-4, BMP-5, BMP-6, BMP-7/OP-1 and BMP-8/OP-2 is altered by addition, substitution, insertion, inversion, and/or deletion, the polypeptide altered by addition, substitution, insertion, inversion and/or deletion exhibiting essentially the same receptor binding affinity to the ectodomain of BMPR-IA as BMP-2, and being at least 50 % homologous thereto, the variant comprising the sequence of SEQ ID NO: 3 or 4.

27. Use of a polypeptide variant according to claim 26, wherein the altered polypeptide is at least 90 % homologous to BMP-2.

## Revendications

1. Variante d'un polypeptide qui est choisi parmi des membres de la superfamille TGFβ, **caractérisée en ce que** la variante de polypeptide a une affinité accrue à l'héparine, au moins un oligopeptide étant ajouté et/ou inséré dans la séquence d'acides aminés du polypeptide choisi parmi des membres de la superfamille TGFβ, et/ou au moins une séquence d'oligopeptide naturellement présente dans la séquence d'acides aminés du polypeptide étant remplacée par un oligopeptide, l'oligopeptide comprenant la séquence d'acides aminés RKRA (SEQ ID n° 3) ou RKRAKHKQ (SEQ ID n° 4).

2. Variante de polypeptide selon la revendication 1, **caractérisée en ce qu'**une à quatre copies de l'oligopeptide sont insérées dans une à quatre positions dans le polypeptide.

3. Variante de polypeptide selon l'une des revendications 1 à 2, **caractérisée en ce que** l'oligopeptide est ajouté à la terminaison N et/ou est inséré dans la zone terminale N et/ou remplace une partie de la zone terminale N.

4. Variante de polypeptide selon l'une des revendications 1 à 3, **caractérisée en ce que** la séquence d'acides aminés de la variante de polypeptide comprend en plus à la terminaison N une séquence déterminante pour l'expression recombinante, la séquence déterminante pour l'expression recombinante étant M ou MZ, M étant la méthionine et Z un ou plusieurs acides aminés quelconques.

5. Variante de polypeptide selon l'une des revendications 1 à 4, **caractérisée en ce que** la variante de polypeptide comprend aussi un His-Tag.

6. Variante de polypeptide selon l'une des revendications 1 à 5, **caractérisée en ce que** le polypeptide choisi parmi BMP-2, BMP-4, BMP-5, BMP-6, BMP-7/OP-1 et BMP-8/OP-2 est modifié par addition, substitution, insertion, inversion et/ou délétion, le polypeptide modifié par addition, substitution, insertion, inversion et/ou délétion ayant globalement la même affinité réceptrice pour l'ectodomaine de BNPR-IA que BMP-2 et étant au moins 50 % homologue à celui-ci et la variante comprenant la séquence SEQ ID n° 3 ou n° 4.

7. Variante de polypeptide selon l'une des revendications 1 à 6, **caractérisée en ce que** le polypeptide est BMP-2, BMP-4, BMP-5, BMP-6, BMP-7/OP-1 ou BMP-8/OP-2.

8. Variante de polypeptide selon l'une des revendications 1 à 7, **caractérisée en ce que** l'oligopeptide est inséré avant le noeud cystine.

9. Variante de polypeptide selon l'une des revendications 7 à 8, **caractérisée en ce que** la variante de polypeptide a la séquence d'acides aminés SEQ ID n° 5 (T3) ou SEQ ID n° 6 (T4).

10. Variante de polypeptide selon l'une des revendications 1 à 9, **caractérisée en ce que** la variante de polypeptide est un polypeptide, un oligopeptide ou un peptide dimérique de la variante de polypeptide selon l'une des revendications 1 à 9.

11. Molécule d'acide nucléique, comprenant une séquence d'acide nucléique codant une variante de polypeptide selon l'une des revendications 1 à 10.

12. Molécule d'acide nucléique selon la revendication 11, **caractérisée en ce que** la séquence d'acide nucléique est dérivée d'un ADN ou ADNc génomique ou est un ADN synthétique.

13. Molécule d'acide nucléique selon l'une des revendications 11 ou 12, comprenant en plus un promoteur destiné au contrôle d'expression, la séquence d'acide nucléique qui code une variante de polypeptide étant sous le contrôle du promoteur.

14. Molécule d'acide nucléique selon l'une des revendications 11 à 13, la molécule d'acide nucléique contenant au moins une partie d'un vecteur.

15. Cellule hôte, contenant une molécule d'acide nucléique selon l'une des revendications 11 à 14, la cellule hôte étant une cellule procaryotique ou eucaryotique destinée à l'expression de la molécule d'acide nucléique.

16. Procédé de fabrication d'une variante de polypeptide ayant une affinité accrue à l'héparine selon la revendication 1, comprenant
- l'addition d'au moins un oligopeptide qui contient une séquence d'acides aminés choisie parmi : SEQ ID n° 3 et SEQ ID n° 4 à la séquence d'acides aminés d'un polypeptide ;
- et/ou l'insertion d'au moins un oligopeptide qui contient une séquence d'acides aminés choisie parmi : SEQ ID n° 3 et SEQ ID n° 4 dans la séquence d'acides aminés d'un polypeptide ;
- et/ou la substitution d'un oligopeptide qui contient une séquence d'acides aminés choisie parmi : SEQ ID n° 3 et SEQ ID n° 4 à au moins une séquence d'oligopeptide naturellement présente dans la séquence d'acides aminés d'un polypeptide.

17. Procédé selon la revendication 16, **caractérisé en ce que** le procédé comprend un procédé de synthèse chimique et/ou enzymatique.

18. Procédé selon l'une des revendications 16 ou 17, **caractérisé en ce que** le procédé comprend des procédés du génie génétique.

19. Procédé selon l'une des revendications 16 à 18, **caractérisé en ce que** le procédé comprend :
a) mutagenèse in vitro d'un acide nucléique codant un polypeptide de telle sorte que (i) l'on ajoute à l'acide nucléique codant le polypeptide au moins un acide nucléique codant un oligopeptide qui contient une séquence d'aminés choisie parmi SEQ ID n° 3 et n° 4 ; et/ou (ii) l'on insère dans l'acide nucléique codant le polypeptide au moins un acide nucléique codant un oligopeptide qui contient une séquence d'aminés choisie parmi SEQ ID n° 3 et n° 4 ; et/ou (iii) on substitue à au moins une séquence d'acide nucléique naturellement présente dans l'acide nucléique codant le polypeptide une séquence d'acide nucléique codant un oligopeptide qui contient une séquence d'aminés choisie parmi SEQ ID n° 3 et n° 4 ;
b) clonage de l'acide nucléique muté dans un vecteur d'expression approprié ;
c) transformation / transfection d'une cellule hôte appropriée avec le vecteur d'expression obtenu ;
d) culture de la cellule hôte transformée / transfectée dans des conditions convenant à l'expression ;
e) isolation et éventuellement renaturation de la variante de polypeptide exprimée.

20. Procédé selon l'une des revendications 16 à 19, **caractérisé en ce que** le procédé est mis en oeuvre dans une cellule hôte procaryotique, de préférence E. coli.

21. Procédé selon l'une des revendications 16 à 19, **caractérisé en ce que** le procédé est mis en oeuvre dans une cellule hôte eucaryotique, de préférence une cellule de levure, de plante, d'insecte, CHO ou COS.

22. Composé pharmaceutique, comprenant une variante de polypeptide selon l'une des revendications 1 à 10 et éventuellement des additifs physiologiquement acceptables.

23. Utilisation d'une variante de polypeptide selon l'une des revendications 1 à 10 pour la fabrication d'un composé pharmaceutique destiné à stimuler l'ostéogenèse ou la cicatrisation ou à traiter une inflammation ou un cancer.

24. Composé d'ostéoinduction, comprenant une variante de polypeptide selon l'une des revendications 1 à 10 et un support choisi parmi l'héparine, l'hydroxylapatite, l'acide hyaluronique, des polymères synthétiques et des collagènes.

25. Matrice ostéoinductive, **caractérisée en ce qu'**elle contient de l'héparine ou des substances analogues à l'héparine ou est recouverte de celles-ci et **en ce que** des variantes de polypeptide selon l'une des revendications 1 à 10 sont adsorbées sur l'héparine ou sur les substances analogues à l'héparine.

26. Utilisation d'une variante de polypeptide selon l'une des revendications 1 à 5 pour la fabrication d'un composé pharmaceutique destiné à stimuler l'ostéogenèse ou la cicatrisation ou à traiter une inflammation ou un cancer, le polypeptide choisi parmi BMP-2, BMP-4, BMP-5, BMP-6, BMP-7/OP-1 et BMP-8/OP-2 étant modifié par addition, substitution, insertion, inversion et/ou délétion, le polypeptide modifié par addition, substitution, insertion, inversion et/ou délétion ayant globalement la même affinité réceptrice pour l'ectodomaine de BNPR-IA que BMP-2 et étant au moins 50 % homologue à celui-ci et la variante comprenant la séquence SEQ ID n° 3 ou n° 4.

27. Utilisation d'une variante de polypeptide selon la revendication 26, le polypeptide modifié étant au moins 90 % homologue à BMP-2.


### SEQUENZPROTOKOLL

<110> Sebald, Walter
<120> Polypeptidvarianten mit erhöhter Heparin-Bindungsfähigkeit
<130> PCT1126-01996
<140>
   <141>
<150> DE 199 06 096.7
   <151> 1999-02-13
<160> 12
<170> PatentIn Ver. 2.1
<210> 1
   <211> 6
   <212> PRT
   <213> Künstliche Sequenz
<220>
   <221> MUTAGEN
   <222> (1)
   <223> K, R oder H
<220>
   <221> MUTAGEN
   <222> (2)
   <223> K, R oder H
<220>
   <221> MUTAGEN
   <222> (3)
   <223> K, R, H oder keine Aminosäure
<220>
   <221> MUTAGEN
   <222> (4)
   <223> kein K, R, H, sonst beliebige Aminosäure
<220>
   <221> MUTAGEN
   <222> (5)
   <223> kein K, R, H, sonst beliebige oder keine Aminosäure
<220>
   <221> MUTAGEN
   <222> (6)
   <223> kein K, R, H, sonst beliebige oder keine Aminosäure
<220>
   <223> Beschreibung der künstlichen Sequenz: Künstliche Sequenz
<400> 1
   Xaa Xaa Xaa Xaa Xaa Xaa
    1               5
<210> 2
   <211> 6
   <212> PRT
   <213> Künstliche Sequenz
<220>
   <223> Beschreibung der künstlichen Sequenz: Künstliche Sequenz
<220>
   <221> MUTAGEN
   <222> (1)
   <223> K, R oder H
<220>
   <221> MUTAGEN
   <222> (2)
   <223> kein K, R, H, sonst beliebige Aminosäure
<220>
   <221> MUTAGEN
   <222> (3)
   <223> K, R oder H
<220>
   <221> MUTAGEN
   <222> (4)
   <223> kein K, R, H, sonst beliebige Aminosäure
<220>
   <221> MUTAGEN
   <222> (5)
   <223> kein K, R, H, sonst beliebige oder keine Aminosäure
<220>
   <221> MUTAGEN
   <222> (6)
   <223> kein K, R, H, sonst beliebige oder keine Aminosäure
<400> 2
   Xaa Xaa Xaa Xaa Xaa Xaa
    1               5
<210> 3
   <211> 4
   <212> PRT
   <213> Künstliche Sequenz
<220>
   <223> Beschreibung der künstlichen Sequenz:
   Heparin-Bindungssequenz
<400> 3
   Arg Lys Arg Ala
     1
<210> 4
   <211> 8
   <212> PRT
   <213> Künstliche Sequenz
<220>
   <223> Beschreibung der künstlichen Sequenz: Heparin-Bindungssequenz
<400> 4
   Arg Lys Arg Ala Lys His Lys Gln
     1               5
<210> 5
   <211> 120
   <212> PRT
   <213> Künstliche Sequenz
<220>
   <223> Beschreibung der künstlichen Sequenz: T3
<400> 5
   Met Ala Gln Ala Lys His Lys Gln Arg Lys Arg Ala Arg Lys Arg Leu
   1               5                   10                  15
   Lys Ser Ser Cys Lys Arg His Pro Leu Tyr Val Asp Phe Ser Asp Val
               20                  25                  30
   Gly Trp Asn Asp Trp Ile Val Ala Pro Pro Gly Tyr His Ala Phe Tyr
           35                  40                  45
   Cys His Gly Glu Cys Pro Phe Pro Leu Ala Asp His Leu Asn Ser Thr
       50                  55                  60
   Asn His Ala Ile Val Gln Thr Leu Val Asn Ser Val Asn Ser Lys Ile
   65                  70                  75                  80
   Pro Lys Ala Cys Cys Val Pro Thr Glu Leu Ser Ala Ile Ser Met Leu
                   85                  90                  95
   Tyr Leu Asp Glu Asn Glu Lys Val Val Leu Lys Asn Tyr Gln Asp Met
               100                 105                 110
   Val Val Glu Gly Cys Gly Cys Arg
           115                 120
<210> 6
   <211> 124
   <212> PRT
   <213> Künstliche Sequenz
<220>
   <223> Beschreibung der künstlichen Sequenz:T4
<400> 6
   Met Ala Gln Ala Lys His Lys Gln Arg Lys Arg Ala Lys His Lys Gln
    1               5                  10                  15
   Arg Lys Arg Leu Lys Ser Ser Cys Lys Arg His Pro Leu Tyr Val Asp
               20                  25                  30
   Phe Ser Asp Val Gly Trp Asn Asp Trp Ile Val Ala Pro Pro Gly Tyr
           35                  40                  45
   His Ala Phe Tyr Cys His Gly Glu Cys Pro Phe Pro Leu Ala Asp His
       50                  55                  60
   Leu Asn Ser Thr Asn His Ala Ile Val Gln Thr Leu Val Asn Ser Val
   65                  70                  75                  80
   Asn Ser Lys Ile Pro Lys Ala Cys Cys Val Pro Thr Glu Leu Ser Ala
                   85                  90                  95
   Ile Ser Met Leu Tyr Leu Asp Glu Asn Glu Lys Val Val Leu Lys Asn
               100                 105                 110
   Tyr Gln Asp Met Val Val Glu Gly Cys Gly Cys Arg
           115                 120
<210> 7
   <211> 374
   <212> DNA
   <213> Künstliche Sequenz
<220>
   <223> Beschreibung der künstlichen Sequenz:T3 (Nukleinsäuresequenz)
<400> 7
   ccatggctca agccaaacac aaacagcgga aacgcgctcg taaacgtctt aagtccagct 60
   gtaagagaca ccctttgtac gtggacttca gtgacgtggg gtggaatgac tggattgtgg 120
   ctcccccggg gtatcacgcc ttttactgcc acggagaatg cccttttcct ctggctgatc 180
   atctgaactc cactaatcat gccattgttc agacgttggt caactctgtt aactctaaga 240
   ttcctaaggc atgctgtgtc ccgacagaac tcagtgctat ctcgatgctg taccttgacg 300
   agaatgaaaa ggttgtatta aagaactatc aggacatggt tgtggagggt tgtgggtgtc 360
   gctagtaagg atcc                                                   374
<210> 8
   <211> 386
   <212> DNA
   <213> Künstliche Sequenz
<220>
   <223> Beschreibung der künstlichen Sequenz: T4 (Nukleinsäuresequenz)
<400> 8
   ccatggctca agccaaacac aaacagcgga aacgcgctaa gcataagcaa cgtaagcgtc 60
   ttaagtccag ctgtaagaga caccctttgt acgtggactt cagtgacgtg gggtggaatg 120
   actggattgt ggctcccccg gggtatcacg ccttttactg ccacggagaa tgcccttttc 180
   ctctggctga tcatctgaac tccactaatc atgccattgt tcagacgttg gtcaactctg 240
   ttaactctaa gattcctaag gcatgctgtg tcccgacaga actcagtgct atctcgatgc 300
   tgtaccttga cgagaatgaa aaggttgtat taaagaacta tcaggacatg gttgtggagg 360
   gttgtgggtg tcgctagtaa ggatcc                                      386
<210> 9
   <211> 47
   <212> DNA
   <213> Künstliche Sequenz
<220>
   <223> Beschreibung der künstlichen Sequenz: künstlich
<400> 9
   catggctcaa gccaaacaca aacagcggaa acgcgctcgt aaacgtc 47
<210> 10
   <211> 47
   <212> DNA
   <213> Künstliche Sequenz
<220>
   <223> Beschreibung der künstlichen Sequenz: künstlich
<400> 10
   ttaagacgtt tacgagcgcg tttccgctgt ttgtgtttgg cttgagc 47
<210> 11
   <211> 59
   <212> DNA
   <213> Künstliche Sequenz
<220>
   <223> Beschreibung der künstlichen Sequenz: künstlich
<400> 11
   catggctcaa gccaaacaca aacagcggaa acgcgctaag cataagcaac gtaagcgtc 59
<210> 12
   <211> 59
   <212> DNA
   <213> Künstliche Sequenz
<220>
   <223> Beschreibung der künstlichen Sequenz: künstlich
<400> 12
   ttaagacgct tacgttgctt atgcttagcg cgtttccgct gtttgtgttt ggcttgagc 59